(19) Europäisches Patentamt
European Patent Office
Office européen des brevets

(11) EP 3 202 418 B1

(12) **EUROPEAN PATENT SPECIFICATION**

(45) Date of publication and mention of the grant of the patent:
**23.08.2023 Bulletin 2023/34**

(21) Application number: **16183212.6**

(22) Date of filing: **30.04.2008**

(51) International Patent Classification (IPC):
***A61K 39/00** (2006.01)* ***C07K 16/28** (2006.01)*
***C12N 5/0783** (2010.01)*

(52) Cooperative Patent Classification (CPC):
**C12N 5/0636; A61P 29/00; A61P 37/00; A61P 37/06; C07K 16/2803;** A61K 2039/505; C07K 2317/732; C07K 2317/734; C07K 2317/92; C12N 2501/998

(54) **CYTOTOXIC ANTI-LAG-3 MONOCLONAL ANTIBODY AND ITS USE IN THE TREATMENT OR PREVENTION OF ORGAN TRANSPLANT REJECTION AND AUTOIMMUNE DISEASE**

ZYTOTOXISCHER MONOKLONALER ANTI-LAG-3-ANTIKÖRPER UND SEINE VERWENDUNG BEI DER BEHANDLUNG UND VORBEUGUNG VON ORGANTRANSPLANTATABSTOSSUNG UND AUTOIMMUNERKRANKUNGEN

ANTICORPS MONOCLONAL ANTI-LAG-3 CYTOTOXIQUE ET SON UTILISATION DANS LE TRAITEMENT OU LA PRÉVENTION D'UN REJET DU GREFFON D'ORGANE ET DE MALADIES AUTO-IMMUNES

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **30.04.2007 EP 07290545**

(43) Date of publication of application:
**09.08.2017 Bulletin 2017/32**

(62) Document number(s) of the earlier application(s) in accordance with Art. 76 EPC:
**08750865.1 / 2 142 210**

(73) Proprietors:
- **Immutep**
  **91893 Orsay Cedex (FR)**
- **INSERM (Institut National de la Santé et de la Recherche Scientifique)**
  **75654 Paris Cedex 13 (FR)**

(72) Inventors:
- **TRIEBEL, Frédéric**
  **78000 Versailles (FR)**
- **VANHOVE, Bernard**
  **44400 Rezé (FR)**
- **HAUDEBOURG, Thomas**
  **44000 Nantes (FR)**

(74) Representative: **Lee, Alison Lesley**
**GlaxoSmithKline**
**Global Patents (CN925.1)**
**980 Great West Road**
**Brentford, Middlesex TW8 9GS (GB)**

(56) References cited:
**WO-A-2004/078928** **WO-A1-2005/103086**
**US-A- 5 976 877**

- **MACON-LEMAITRE LAETITIA ET AL: "The negative regulatory function of the lymphocyte-activation gene-3 co-receptor (CD223) on human T cells", IMMUNOLOGY, vol. 115, no. 2, June 2005 (2005-06), pages 170-178, XP002453818, ISSN: 0019-2805**
- **MONK NICOLA J ET AL: "Fc-dependent depletion of activated T cells occurs through CD40L-specific antibody rather than costimulation blockade.", NATURE MEDICINE, vol. 9, no. 10, October 2003 (2003-10), pages 1275-1280, XP002453819, ISSN: 1078-8956**
- **HAUDEBOURG THOMAS ET AL: "Depletion of LAG-3 positive cells in cardiac allograft reveals their role in rejection and tolerance", TRANSPLANTATION, WILLIAMS AND WILKINS, GB, vol. 84, no. 11, 1 December 2007 (2007-12-01), pages 1500-1506, XP002495209, ISSN: 0041-1337**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- DRAKE C G ET AL: "Blocking the regulatory T cell molecule LAG-3 augments in vivo anti-tumor immunity in an autochthonous model of prostate cancer", JOURNAL OF CLINICAL ONCOLOGY, AMERICAN SOCIETY OF CLINICAL ONCOLOGY, US, vol. 24, no. 18, suppl, 20 June 2006 (2006-06-20), page 2573, XP009138507, ISSN: 0732-183X
- HUANG CHING-TAI ET AL: "Role of LAG-3 in regulatory T cells", IMMUN, CELL PRESS, US, vol. 21, no. 4, 1 October 2004 (2004-10-01), pages 503-513, XP009138514, ISSN: 1074-7613, DOI: 10.1016/J.IMMUNI.2004.08.010 [retrieved on 2004-10-19]
- MALGORZATA KISIELOW ET AL: "Expression of lymphocyte activation gene 3 (LAG-3) on B cells is induced by T cells", EUROPEAN JOURNAL OF IMMUNOLOGY, vol. 35, no. 7, 1 July 2005 (2005-07-01), pages 2081-2088, XP055162755, ISSN: 0014-2980, DOI: 10.1002/eji.200526090
- VELDERS M P ET AL: "THE IMPACT OF ANTIGEN DENSITY AND ANTIBODY AFFINITY ON ANTIBODY-DEPENDENT CELLULAR CYTOTOXICITY: RELEVANCE FOR IMMUNOTHERAPY OF CARCINOMAS", BRITISH JOURNAL OF CA, NATURE PUBLISHING GROUP, GB, vol. 78, no. 4, 1 August 1998 (1998-08-01) , pages 478-483, XP009027871, ISSN: 0007-0920
- IYAD JAMALI ET AL: "KINETICS OF ANTI-CD4-INDUCED T HELPER CELL DEPLETION AND INHIBITION OF FUNCTION Activation of T Cells by the CD3 Pathway Inhibits Anti-CD4-Mediated T Cell Elimination and Down-Regulation of Cell Surface CD4", THE JOURNAL OF IMMUNOLOGY, vol. 148, no. 6, 15 March 1992 (1992-03-15), pages 1613-1619, XP055213914, ISSN: 0022-1767
- N. POIRIER ET AL: "Antibody-mediated depletion of lymphocyte-activation gene-3 (LAG-3+)-activated T lymphocytes prevents delayed-type hypersensitivity in non-human primates", CLINICAL & EXPERIMENTAL IMMUNOLOGY, vol. 164, no. 2, 1 May 2011 (2011-05-01), pages 265-274, XP055055512, ISSN: 0009-9104, DOI: 10.1111/j.1365-2249.2011.04329.x
- UCHIDA J ET AL: "The innate mononuclear phagocyte network depletes B lymphocytes through Fc receptor-dependent mechanisms during anti-CD20 antibody immunotherapy", THE JOURNAL OF EXPERIMENTAL MEDICINE, ROCKEFELLER UNIVERSITY PRESS, US, vol. 199, no. 12, 21 June 2004 (2004-06-21), pages 1659-1669, XP003002436, ISSN: 0022-1007, DOI: 10.1084/JEM.20040119
- YOUSAF N ET AL: "TARGETING BEHAVIOR OF RAT MONOCLONAL IGG ANTIBODIES IN-VIVO ROLE OF ANTIBODY ISOTYPE SPECIFICITY AND THE TARGET CELL ANTIGEN DENSITY", EUROPEAN JOURNAL OF IMMUNOLOGY, WILEY VCH, WEINHEIM, vol. 21, no. 4, 1 January 1991 (1991-01-01), pages 943-950, XP009138554, ISSN: 0014-2980, DOI: 10.1002/EJI.1830210413
- CHAN L A ET AL: "Variable region domain exchange in human IgGs promotes antibody complex formation with accompanying structural changes and altered effector functions", MOLECULAR IMMUNOLOGY, PERGAMON, GB, vol. 41, no. 5, 1 July 2004 (2004-07-01), pages 527-538, XP002519620, ISSN: 0161-5890, DOI: 10.1016/J.MOLIMM.2004.03.034
- TEELING JESSICA L ET AL: "The biological activity of human CD20 monoclonal antibodies is linked to unique epitopes on CD20", THE JOURNAL OF IMMUNOLOGY, THE AMERICAN ASSOCIATION OF IMMUNOLOGISTS, US, vol. 177, no. 1, 1 July 2006 (2006-07-01), pages 362-371, XP002453762, ISSN: 0022-1767
- DATABASE BIOSIS [Online] BIOSCIENCES INFORMATION SERVICE, PHILADELPHIA, PA, US; 1991, YOUSAF N ET AL: "TARGETING BEHAVIOR OF RAT MONOCLONAL IGG ANTIBODIES IN-VIVO ROLE OF ANTIBODY ISOTYPE SPECIFICITY AND THE TARGET CELL ANTIGEN DENSITY", Database accession no. PREV199192008654 & YOUSAF N ET AL: "TARGETING BEHAVIOR OF RAT MONOCLONAL IGG ANTIBODIES IN-VIVO ROLE OF ANTIBODY ISOTYPE SPECIFICITY AND THE TARGET CELL ANTIGEN DENSITY", EUROPEAN JOURNAL OF IMMUNOLOGY, vol. 21, no. 4, 1991, pages 943-950, ISSN: 0014-2980
- DATABASE BIOSIS [Online] BIOSCIENCES INFORMATION SERVICE, PHILADELPHIA, PA, US; April 1998 (1998-04), HUARD BERTRAND ET AL: "LAG-3 does not define a specific mode of natural killing in human", Database accession no. PREV199800319606 & HUARD BERTRAND ET AL: "LAG-3 does not define a specific mode of natural killing in human", IMMUNOLOGY LETTERS, vol. 61, no. 2-3, April 1998 (1998-04), pages 109-112, ISSN: 0165-2478
- HUARD B ET AL: "LYMPHOCYTE-ACTIVATION GENE 3/MAJOR HISTOCOMPATIBILITY COMPLEX CLASS II INTERACTION MODULATES THE ANTIGENIC RESPONSE OF CD4+ T LYMPHOCYTES", EUROPEAN JOURNAL OF IMMUNOLOGY, WILEY-VCH, HOBOKEN, USA, vol. 24, no. 12, 1 January 1994 (1994-01-01), pages 3216-3221, XP000672156, ISSN: 0014-2980, DOI: 10.1002/EJI.1830241246




(Cont. next page)

- **DATABASE BIOSIS [Online] BIOSCIENCES INFORMATION SERVICE, PHILADELPHIA, PA, US; June 2008 (2008-06), NISHIDA MICHIO ET AL: "Novel humanized anti-CD20 monoclonal antibodies with unique germline VH and VL gene recruitment and potent effector functions", Database accession no. PREV200800463830 & NISHIDA MICHIO ET AL: "Novel humanized anti-CD20 monoclonal antibodies with unique germline VH and VL gene recruitment and potent effector functions", INTERNATIONAL JOURNAL OF ONCOLOGY, vol. 32, no. 6, June 2008 (2008-06), pages 1263-1274, ISSN: 1019-6439**
- **DATABASE BIOSIS [Online] BIOSCIENCES INFORMATION SERVICE, PHILADELPHIA, PA, US; 1988, BINDON C I ET AL: "IMPORTANCE OF ANTIGEN SPECIFICITY FOR COMPLEMENT-MEDIATED LYSIS BY MONOCLONAL ANTIBODIES", Database accession no. PREV198987037999 & BINDON C I ET AL: "IMPORTANCE OF ANTIGEN SPECIFICITY FOR COMPLEMENT-MEDIATED LYSIS BY MONOCLONAL ANTIBODIES", EUROPEAN JOURNAL OF IMMUNOLOGY, vol. 18, no. 10, 1988, pages 1507-1514, ISSN: 0014-2980**


Remarks:

The complete document including Reference Table(s) and the Sequence Listing(s) can be downloaded from the EPO website

## Description

### I - Field of the invention

The present invention is in the field of immunotherapy. More specifically, it relates to the treatment or prevention of organ transplant rejection or for treating autoimmune disease. The invention relates to cytotoxic anti-LAG-3 antibodies or fragments thereof binding to LAG-3 protein and causing depletion of LAG-3$^+$ activated T cells.

### II - Background

Lymphocyte activation gene-3 (LAG-3, CD223) is up-regulated during the early stages of T-cell activation. The present invention is based on the analysis of the effects of cytotoxic antibodies against LAG-3 in acute cardiac allograft rejection (*in vivo* animal studies) and in *in vitro* experiments where selected LAG-3 monoclonal antibodies are efficient at low doses (<0.1 pg/ml) at depleting LAG-3$^+$ activated effector T cells.

Selectively depleting activated T lymphocytes might represent an immunosuppressive induction treatment able to result in the development of regulatory cells supporting a long-term survival of allogeneic organs in mice and primates (1). This has actually been demonstrated with anti-CD40L antibodies that deplete in vivo activated T cells through a Fc-dependent mechanism (2). However, anti-CD40L antibodies also target activated platelets in humans and affect the stability of arterial thrombi (3). Therefore the development of monoclonal antibodies to other molecules specific for T-cell activation has catalyzed attempts to achieve immunosuppression. One such molecule is LAG-3, which engages Class II on dendritic cells (DC) with a high affinity, enabling DC to become activated (4-6). The LAG-3 protein is expressed *in vivo* in activated CD4$^+$ and CD8$^+$ lymphocytes residing in inflamed secondary lymphoid organs or tissues but not in spleen, thymus or blood (7). In addition, LAG-3 can function as a negative regulator of activated human CD4 and CD8 T cells by inhibiting early events in primary activation (8).

WO 2004/078928 discloses methods for the enhancement of the regulatory T cell response, for example by increasing the number of CD223$^+$ cells in a T cell population.

Monk et al, Nature Medicine, vol. 9, no. 10, 14 September 2003 discloses the depletion of activated T cells by anti-CD40L antibodies.

Kisielow et al, European Journal of Immunology, vol. 35, no. 7, 1 July 2005, pages 2081-2088 discloses that expression of LAG-3 on B cells is induced by T cells.

III - Summary of the invention

The present invention provides a cytotoxic anti-LAG-3 monoclonal antibody or fragment thereof for use in treating or preventing organ transplant rejection or treating an autoimmune disease, wherein said antibody or fragment thereof comprises a human IgG1 or IgM or murine IgG2a subclass Fc fragment and a Fab fragment which binds LAG-3 protein, and further wherein said antibody or fragment thereof causes depletion of LAG-3$^+$ activated T cells via complement-dependent cytotoxicity (CDC) and/or antibody-dependent cell cytotoxicity (ADCC).

Antibodies and fragments thereof according to the present invention bind to LAG-3 protein and cause depletion of LAG-3$^+$ activated T cells. Said depletion can be measured by changes in peripheral blood lymphocyte numbers.

The present invention also provides a cytotoxic anti-LAG-3 monoclonal antibody or fragment thereof for use according to the invention, wherein said monoclonal antibody or fragment thereof is capable of causing depletion of more than 30% preferably more than 50% of LAG-3$^+$ activated T cells.

In addition the present invention provides a cytotoxic anti-LAG-3 monoclonal antibody or fragment thereof for use according to the invention, wherein the antibody or fragment thereof wherein said monoclonal antibody or fragment thereof exhibits (i) more than 50% of maximal CDC activity at a mAb concentration of less than 0.1 pg/ml or (ii) more than 50% of maximal ADCC activity at a mAb concentration of less than 0.1 ug/ml. The scope of the invention is defined by the appended claims.

Any references in the description to methods of treatment refer to the compounds, pharmaceutical compositions and medicaments of the present invention for use in a method for treatment of the human (or animal) body by therapy (or for diagnosis).

### IV - Description of the figures

Figure 1: LAG-3 mRNA expression in cardiac allograft (A), in the spleen (B) and in lymph nodes (C). Expression of LAG-3 mRNA in heart grafts at day 5 was measured by quantitative RT-PCR and compared with housekeeping HPRT transcripts expression. Rejection: allograft without treatment. Syngenic: isograft. Tolerant: allograft in recipients receiving a tolerogenic (CsA+anti-CD28 antibodies) treatment. **: p<0.05 for syngenic and tolerant vs. rejection.

Figure 2: Characterization of anti-LAG-3 antibodies in a complement-dependant cytotoxicity assay. ConA-stimulated target cells were labeled with $^{51}Cr$ and mixed with rabbit complement and anti-LAG-3 (full line) or preimmune (dotted line) serum at the indicated dilution. % cytotoxic activity is calculated as follows: (CPM of the assay - spontaneous CPM release)/ maximum released CPM obtained after cell lysis.

Figure 3: In vitro depleting activity of anti-LAG-3 antibodies. T cells from the spleen were activated for 48h with Con A to induce expression of LAG-3 and labeled with CFSE. 105 cells were injected i.v. to recipients that had been irradiated (4.5 Gy) 3 days before. Twenty-four hours after injection, recipients were sacrificed and the presence of $CFSE^+$ cells in the $CD4^+$ and $CD8^+$ compartments of the spleen analyzed by flow cytometry.

Figure 4: Heart graft survival after anti-LAG-3 antibodies administration. Lew.1A recipients of fully allogeneic (class I and II mismatch) Lew.1W hearts were treated by injections on days 0 and 3 of 200 μl (dashed line) or 600 μl (full line) rabbit anti-LAG-3 serum or of 600 μl pre-immune serum (dotted line). Graft survival was evaluated by daily evaluation of heartbeat. P<0.002 for 600 μl rabbit anti-LAG-3 serum vs. pre-immune serum.

Figure 5: Analysis of Graft infiltrating cells (GICs). GICs were extracted from cardiac allografts on day 5 from control-treated or anti-LAG-3 antibodies treated recipients. Cells were counted and analyzed by flow cytometry for the expression of LAG-3. White bars: total number of GICs. Black bars: LAG-$3^+$ GICs measured by flow cytometry (p< 0.01). Total RNA was also extracted from GICs and messenger for INFγ were quantified by qPCR, relative to HPRT expression level (dashed bars; p< 0.05).

Figure 6: Comparison of A9H12 binding with the reference LAG-3 specific 17B4 mAb on LAG-$3^+$ CHO and activated human T cells.

A) hLAG-3-transfected CHO were dissociated from culture plastic using Versene buffer containing cation-chelating agent, incubated with indicated concentrations of A9H12 or 17B4 mAbs for 30 min at 4°C, washed and then incubated with a FITC-conjugated goat anti-mouse IgG+M (H+L) secondary antibody (5 ug/ml, Coulter) for 30 minutes at 4°C. After washing, cells were analysed by flow cytometry using a FACSCanto (BD Biosciences) and means of fluorescence intensity were plotted as a function of antibody concentration.

B) PBMCs from a healthy volunteer were stimulated for 2 days with SEB (1 ug/ml, Sigma Aldrich) to induce the expression of LAG-3 on T cells. PBMCs were stained as above. Data represent a weighted percentage, calculated as the percentage of LAG-$3^+$ cells in PBMCs x mean of fluorescence intensity of the LAG-$3^+$ cells, plotted as a function of antibody concentration.

Figure 7: Complement-Dependent Cytotoxicity induced by A9H12 LAG-3 mAb.

A) hLAG-3-transfected and wild type CHO cells were labelled with FITC-conjugated anti-LAG-3 mAb (17B4) and the expression of LAG-3 on cell surface was analysed by flow cytometry using a FACSCanto. The histogram plots represent the mean fluorescence intensity of wt CHO (gray) and LAG-$3^+$ CHO (dark).

B) *hLAG-3*-transfected and wt CHO cells were washed in complete medium (MEM supplemented with 10 % heat inactivated Foetal Calf Serum, FCS) and incubated with 0.1 pg/ml of A9H12 LAG-3 mAb or mIgG2a isotype-control mAb (Southern Biotechnologies) in complete medium for 30 min at 4 °C. Cells were then washed and incubated in complete medium (-Complement) or in MEM supplemented with 10 % of freshly resuspended rabbit serum (Cerdalane Inc.) (+Complement) for 1 hour at 37°C. After washing, cells were stained with 7-AAD (Coulter Inc.) for 15 minutes at room temperature and immediately analysed by flow cytometry to determined the percentage of 7-AAD-positive cells corresponding to dead cells. Data represent the percentage of dead cells in each condition on hLAG-3-transfected and wt CHO cells as indicated.

C) LAG-$3^+$ CHO cells were incubated with indicated concentrations of A9H12 LAG-3 mAb for 30 min at 4°C and then incubated with MEM supplemented with 25 % rabbit serum for 1 hour at 37°C. After washing, cells were stained with 7-AAD (Coulter Inc.) and analysed by flow cytometry. The percentage of specific cytotoxicity is calculated according to the following formula

$$\frac{\text{(Sample Death - Spontaneous Death)}}{\text{(Maximal Death - Spontaneous Death)}} \times 100$$

where Sample Death is the percentage of 7-AAD-positive cells in each condition, Spontaneous Death, the percentage of 7-AAD-positive cells without mAb and Maximal Death, the percentage of 7-AAD-positive cells with 10 pg/ml mAb.

D) LAG-$3^+$ CHO cells were incubated with 0.1 pg/ml A9H12, 17B4 or 31G11 LAG-3 mAb or with their corresponding isotype controls (IgG2a,IgG1 or IgM, respectively) for 30 min at 4°C and then incubated with MEM supplemented with 25 % rabbit serum for 1 hour at 37°C. Specific cytotoxicity was determined as above with a Maximal Death corresponding to 10 pg/ml A9H12 (left panel) and 0.1 pg/ml A9H12 (right panel).

E) PBMCs were stimulated with SEB (1 pg/ml) to induce LAG-3 expression on T cells and then used as target cells in the CDC assay in the presence of A9H12 or 31G11 LAG-3 mAb or their isotype controls. After staining cells with fluorochrome-conjugated CD3, CD4, CD8, CD25 and 17B4, the percentage of 7-AAD-positive cells was analysed on the indicated T cell subpopulations. Data represent the percentage of dead cells in each population (with spontaneous death in the absence of mAb being subtracted).

Figure 8: Antibody-Dependent Cell-mediated Cytotoxicity induced by A9H12 LAG-3 mAb

A) Effector cells (PBMCs) were stimulated with IL-2 (100 IU/ml, BD Biosciences) for 1 day. Target cells (*hLAG-3*-transfected CHO cells) were labelled with CFSE (a fluorescent vital dye) and incubated with 1 pg/ml A9H12, mIgG2a, 17B4 or mIgG1 for 20 min at room temperature. Effector cells and target cells were then mixed at indicated E:T ratios (E:T, Effector:Target) and incubated for 16 hours at 37°C. Nonadherent and adherent cells were harvested using Versene reagent, stained with 7-AAD and immediately analysed by flow cytometry to determine the percentage of 7-AAD-positive cells in the CFSE-positive population. Data represent the percentage of dead cells, with the non-specific cell death in the presence of the isotype control being subtracted.

B) CFSE-labelled wild-type or LAG-$3^+$ CHO target cells were incubated with indicated concentrations of A9H12 or mIgG2a and IL-2-stimulated PBMCs were added at a 50:1 E:T ratio and incubated for 16 hours at 37°C. Cell death was analysed as above and data represent the percentage of dead cells in CFSE-positive cells in the presence of A9H12 or its isotype-matched IgG2a control mAb.

Figure 9: Incidence of arthritis (percentage of mice that developed CIA)
Male DBA/1 mice (n=22) were injected i.d. with bovine type II collagen (200 µg) emulsified in CFA containing 250 µg *M. tuberculosis.*

Figure 10: Construction of the chimeric IMP731 therapeutic antibody.

Figure 11: Expression plasmids for the light (panel A) and heavy (panel B) IMP731 chains.

Figure 12: Final bi-cistronic plasmid construction used for the stable transfection of CHO cells.

Figure 13: IMP731 binding on LAG-3+ CHO and activated human T cells

A) hLAG-3-transfected CHO were dissociated from culture plastic using Versene buffer containing cation-chelating agent, incubated with indicated concentrations of IMP731 Ab or its isotype control hIgG1 (Chemicon) for 30 min at 4°C, washed and then incubated with a FITC-conjugated $F(ab')_2$ goat anti-human IgG1 secondary antibody (5 ug/ml, SBA) for 30 minutes at 4°C. After washing, cells were analysed by flow cytometry using a FACSCanto (BD Biosciences) and the means of fluorescence intensity were plotted as a function of antibody concentration.

B) PBMCs from a healthy volunteer were stimulated for 2 days with SEB (1 µg/ml, Sigma Aldrich) to induce the expression of LAG-3 on T cells. PBMCs were stained as above. Data represent a weighted percentage, calculated as the percentage of LAG-$3^+$ cells in PBMCs x mean of fluorescence intensity of the LAG-$3^+$ cells, plotted as a function of antibody concentration.

Figure 14: Complement-Dependent Cytotoxicity induced by IMP731 LAG-3 mAb
hLAG-3-transfected CHO cells were incubated with 1 pg/ml of IMP731 Ab or hIgG1 isotype-control mAb (Chemicon) in complete medium (MEM supplemented with 10 % heat inactivated Foetal Calf Serum, FCS) for 30 min at 4 °C. Cells were then washed and incubated in complete medium (without Complement) or in MEM supplemented with 25 % of freshly resuspended rabbit serum (Cerdalane Inc.) (with Complement) for 1 hour at 37°C. After washing, cells were stained with 7-AAD (BD Biosciences) for 15 minutes at room temperature and immediately analysed by flow cytometry to determine the percentage of 7-AAD-positive cells corresponding to dead cells. Data represent the percentage of dead cells in each condition as indicated.

Figure 15: Antibody-Dependent Cell-mediated Cytotoxicity induced by IMP731.

A) Effector cells (PBMCs) were stimulated with IL-2 (100 IU/ml, BD Biosciences) for 1 day. Target cells (*hLAG-3*-transfected CHO cells) were labelled with CFSE (a fluorescent vital dye) and incubated with 1 pg/ml IMP731 or hIgG1 for 10 min at room temperature. Effector cells and target cells were then mixed at indicated E:T ratios (E:T, Effector:Target) and incubated for 16 hours at 37°C. Cells were stained with 7-AAD and immediately analysed by flow cytometry to determine the percentage of 7-AAD-positive cells in the CFSE-positive population. Data represent the percentage of dead cells.

B) CFSE-labelled LAG-$3^+$ CHO target cells were incubated with indicated concentrations of IMP731 or hIgG1 and IL-2-stimulated PBMCs were added at a 50:1 E:T ratio and incubated for 16 hours at 37°C. Cell death was analysed as above in CFSE-positive population. The percentage of specific cytotoxicity, calculated according to the following formula

$$\frac{(\text{Sample Death} - \text{Spontaneous Death}) \times 100}{(\text{Maximal Death} - \text{Spontaneous Death})}$$

where Sample Death is the percentage of 7-AAD-positive cells in each condition, Spontaneous Death, the percentage of 7-AAD-positive cells without Ab and Maximal Death, the percentage of 7-AAD-positive cells with 1 pg/ml IMP731

C) Effector cells (PBMCs) were stimulated with IL-2 (100 IU/ml, BD Biosciences) for 1 day. Target cells (hLAG-$3^+$ CHO cells or hLAG-$3^-$ CHO cells) were labelled with CFSE (a fluorescent vital dye) and incubated with 1 pg/ml IMP731 or hIgG1 for 10 min at room temperature. Effector cells and target cells were then mixed at indicated E:T ratios (E:T, Effector:Target) and incubated for 16 hours at 37°C. Cells were stained with 7-AAD and immediately analysed by flow cytometry to determine the percentage of 7-AAD-positive cells in the CFSE-positive population. Data represent the percentage of dead cells.

V - Detailed description

In a first aspect the present invention provides a cytotoxic anti-LAG-3 monoclonal antibody or fragment thereof for use in treating or preventing organ transplant rejection or treating an autoimmune disease, wherein said antibody or fragment thereof comprises a human IgG1 or IgM or murine IgG2a subclass Fc fragment and a Fab fragment which binds LAG-3 protein, and further wherein said antibody or fragment thereof causes depletion of LAG-$3^+$ activated T cells via complement-dependent cytotoxicity (CDC) and/or antibody-dependent cell cytotoxicity (ADCC).

Antibodies and fragments thereof according to the present invention bind to LAG-3 protein and cause depletion of LAG-$3^+$ activated T cells. Said depletion can be measured by changes in peripheral blood lymphocyte numbers.

In one embodiment, the present invention provides a cytotoxic anti-LAG-3 monoclonal antibody or fragment thereof for use according to the invention, wherein said monoclonal antibody or fragment thereof is capable of causing depletion of more than 30% preferably more than 50% of LAG-$3^+$ activated T cells.

In another embodiment, the present invention provides a cytotoxic anti-LAG-3 monoclonal antibody or fragment thereof for use according to the invention, wherein said monoclonal antibody or fragment thereof exhibits (i) more than 50% of maximal CDC activity at a mAb concentration of less than 0.1 pg/ml or (ii) more than 50% of maximal ADCC activity at a mAb concentration of less than 0.1 pg/ml.

Lymphocyte activation gene-3 (LAG-3, CD223) is up-regulated during the early stages of T-cell activation. The present invention is based on the analysis of the effects of cytotoxic antibodies against LAG-3 in acute cardiac allograft rejection and in rheumatoid arthritis (*in vivo* animal studies) and in *in vitro* experiments where selected LAG-3 monoclonal antibodies are efficient at low doses (<0.1 pg/ml) at depleting LAG-$3^+$ activated effector T cells.

Fully vascularized heterotopic allogeneic heart transplantation was performed in rats across a full-MHC mismatch barrier (LEW.1W into LEW.1A). Recipients received two injections (day 0 and 3) of antibodies directed to the extraloop domain of LAG-3 or control antibodies. Graft survival, histology, mRNA transcripts and alloreactivity of lymphocytes were tested.

It was first noted that LAG-3 mRNA molecules accumulate in cardiac allografts undergoing rejection, but not in peripheral lymphoid organs. Administration of anti-LAG-3 antibodies inhibited graft infiltration by effectors mononuclear cells and prolonged allograft survival from 6 days in control antibodies-treated recipients to a median of 27 days.

It was found that cells expressing LAG-3 infiltrate rejected heart allografts and that targeting LAG-3 using cytotoxic antibodies as induction monotherapy delays acute rejection by reducing graft infiltration by T cells and monocytes.

Experiments showing that short courses of CD40L antibody therapy could achieve long-term graft survival in mice and primates have been initially interpreted as an effect of costimulation blockade. However, Monk *et al.* (2) showed that much of the efficacy of anti-CD40L therapy derives not from costimulation blockade, but from destruction of activated T cells. The outcome is a selective purging of potentially aggressive T cells that have experienced antigen.

Collagen-induced arthritis (CIA) is a well-described animal model for rheumatoid arthritis. Collagen-induced arthritis is an autoimmune disease inducible in rats, mice and primates by immunization with heterologous type II collagen. The resulting joint pathology resembles rheumatoid arthritis with synovial proliferation, cell infiltration, cartilage erosion and bone resorption in the most severe cases (12).

Using particular immunization protocols, early studies have established a hierarchy of responsiveness to CIA linked to the H-2 haplotype, with H-$2^q$ (e.g. DBA/1 mice) being the most and H-$2^b$ (e.g.C57BL/6 mice) amongst the least responsive strains. However, some studies have shown that responsiveness to CIA may be less restricted by the MHC class II than previously thought and may be just as dependent on immunization conditions (13). The variety of type II collagen (CII) from different species and the preparation of complete Freund's adjuvant (CFA) with different concentrations of *Mycobacterium tuberculosis* were important parameters for arthritis development. Inglis et *al.* have shown that chicken, but not bovine, CII was capable of inducing disease in C57BL/6 mice, with an incidence of 50% to 75%. This is in contrast to DBA/1 mice, in which bovine, mouse and chicken CII all induced disease, with an incidence of 80% to 100%. The phenotype of arthritis was milder in C57BL/6 mice than in DBA/1 mice, with less swelling and a more gradual increase in clinical score (14). Moreover, male mice are frequently preferred for CIA studies, as the incidence of arthritis is somewhat higher in male than in female mice.

In mice, CIA is induced by an i.d. injection of type II collagen (CII) in the presence of CFA, usually followed by an i.p. boost injection of CII, without adjuvant, 21 days later. However, there are reported variations for almost every aspect of the immunization procedure and even in the highly susceptible DBA/1 strain the time of onset, severity and incidence of CIA can be variable (13, 15).

Therapeutic antibodies for the treatment of auto-immune diseases have already been described, like the TNF$\alpha$ mAbs in rheumatoid arthritis. By definition, LAG-3 (Lymphocyte Activation Gene-3) is a marker for recently activated effector T cells. Depleting these effector LAG-3+ T cells will lead to targeted immunosuppression (i.e. only activated T cells are suppressed, not all T cells as with corticoids or cyclosporin). This very specific immunosuppression should lead to higher therapeutic indices compared to classical immunosuppressive agents or to therapeutic antibodies (e.g. Humira, Remicade) or soluble receptors (e.g. Enbrel) blocking TNF$\alpha$. Thus, LAG-3 is a promising target available for a therapeutic depleting mAb approach to eliminate auto-reactive activated effector T cells.

Cytotoxic anti-LAG-3 monoclonal antibodies or fragments thereof for use according to the present invention cause depletion of more than 30% preferably more than 50 % of LAG-3+ activated T cells.

Cytotoxic anti-LAG-3 monoclonal antibodies or fragments thereof for use according to the invention comprise antibodies with a murine IgG2a or a human IgG1 or IgM Fc region giving strong CDC or ADCC properties.

Cytotoxic anti-LAG-3 monoclonal antibodies or fragments thereof for use according to the present invention exhibit (i) more than 50 % of maximal CDC activity at a mAb concentration of less than 0.1 pg/ml and/or (ii) more than 50 % of maximal ADCC activity at a mAb concentration of less than 0.1 pg/ml.

Cytotoxic anti-LAG-3 monoclonal antibodies for use according to the present invention, which cause depletion of LAG-3+ activated T cells, can be produced by methods well known to those skilled in the art.

Antibodies generated against CD223 polypeptides can be obtained by administering, in particular by direct injection, CD223 polypeptides to an animal, preferably a non-human. The antibody so obtained will then bind the CD223 polypeptides itself. In this manner, even a sequence encoding only a fragment of the CD223 polypeptide can be used to generate antibodies binding the whole native CD223 polypeptide.

For preparation of monoclonal antibodies, any technique which provides antibodies produced by continuous cell line cultures can be used. Examples include the hybridoma technique (9), the trioma technique, the human B-cell hybridoma technique (10) .

Techniques described for the production of single chain antibodies (US. Pat. No. 4,946,778) can be readily used to produce single chain antibodies to CD223 polypeptides. Also, transgenic mice may be used to express humanized antibodies to immunogenic CD223 polypeptides.

A first monoclonal antibody suitable for use according to the present invention, called A9H12, is produced by the hybridoma deposited at the CNCM on April 27, 2007 under the access number CNCM I-3755.

A second monoclonal antibody suitable for use according to the present invention, called 31G11, is produced by the hybridoma deposited at the CNCM on April 27, 2007 under the access number CNCM I-3756.

The invention is directed to cytotoxic anti-LAG-3 monoclonal antibodies or fragments thereof for use in treating or preventing organ transplant rejection or for treating an autoimmune disease, wherein said antibody or fragment thereof comprises a human IgG1 or IgM or murine IgG2a subclass Fc fragment and a Fab fragment which binds LAG-3 protein, and further wherein said antibody or fragment thereof causes depletion of LAG-3$^+$ activated T cells via complement-dependent cytotoxicity (CDC) and/or antibody-dependent cell cytotoxicity (ADCC). (ADCC), as defined in the appended claims.

Organ transplant rejection refers to the graft of an organ in an allogenic host. It may be useful for treating organisms suffering from conditions resulting in an abnormally high T-cell population or deleterious T-cell activity, for example graft rejection mediated by host T-cells, graft vs. host disease and T-cell mediated autoimmune and inflammatory diseases such as rheumatoid arthritis, type 1 diabetes, muscular sclerosis, etc.

Auto-immune diseases are diseases in which the subject's own immune system reacts against the subject cells. Auto-immune disease which are amenable to treatments according to the present invention include autoimmune hemolytic anemia, autoimmune thrombocytopenia purpura, Goodpasture's syndrome, pemphigus vulgaris, acute rheumatic fever, mixed essential cryoglobulinemia, systemic lupus erythematosus, insulin-dependent diabetes mellitus, rheumatoid arthritis, Grave's disease, Hashimoto's thyroiditis, myasthenia gravis, and multiple sclerosis.

EXAMPLE 1 : LAG-3-positive cells targeted with cytotoxic antibodies

Material and methods

*Animals and transplantations*

Eight- to 12-week-old male Lewis.1W (LEW.1W, haplotype RT1$^u$) and Lewis.1A (LEW. 1A, haplotype RT1$^a$) congeneic rats (Centre d'Elevage Janvier, Le Genest-Saint-Isle, France), differed in their entire MHC region. Heterotopic LEW.1W heart transplantation was performed as previously described (11). Graft survival was evaluated by palpation through the abdominal wall.

*Anti-LAG-3 antibodies*

A synthetic peptide corresponding to the extraloop domain of the rat LAG-3 protein (NCBI accession nb DQ438937; peptide DQPASIPALDLLQGMPSTRRHPPHR) was linked to ovalbumin and used to immunise two rabbits. Pre-immune and immune sera, collected on day 63 after the 4$^{th}$ immunisation, were assayed by ELISA on immunogen and peptide and by flow cytometry on Con-A activated rat spleen cells. Pre-immune sera were negative in both assays. Pooled immune sera presented a titer (dilution for 50% signal) of 1/60000 by ELISA and of 1/1000 by FACS, and presented a specificity for activated T cells.

*Complement-dependent cytotoxicity assay*

Complement-mediated antibody-dependent cytotoxicity was tested using rabbit sera against Lewis 1A T cells in a $^{51}$Cr release assay. A total of $2\times10^6$ Lewis 1A T cells were labelled with 30 pCi of $^{51}$Cr for 90 min at 37°C in RPMI (GIBCO) with 10% FCS. After three washes, T cells were distributed in 96 V-bottomed plates and incubated with rabbit complement and serial dilutions of heat-inactivated rabbit serum. After 4 h at 37°C, $^{51}$Cr release was measured in the supernatants using a scintillation counter. Specific cytotoxicity was calculated according to the following formula: (experimental release - spontaneous release) $\times$ 100/(maximum release - spontaneous release).

*In vivo antibody-induced cytotoxicity*

Cytotoxic activity of anti-LAG-3 antibodies against LAG-3$^+$ cells was evaluated in vivo. ConA-activated (48h) LEW.1W splenocytes were labelled with the CFSE and transferred ($10^8$ cells) into irradiated (4.5 Gy, day -3) LEW.1A recipients, together with anti-LAG-3 antibodies. On day 1, recipients were sacrificed and the presence of CFSE-positive cells evaluated by flow cytometry in lymphoid organs and in the blood.

*Immunostaining*

Graft samples were embedded in Tissue Tek (OCT Compound, Torrance, CA, USA), snap-frozen in liquid nitrogen, cut into 5 $\mu$m sections and fixed in acetone. Endogenous biotin activity was blocked using the Dako biotin blocking system (Dako, Trappes, France). Sections were then labelled by a three-step indirect immunoperoxidase revelation. The area of each immunoperoxidase-labeled tissue section infiltrated by cells was determined by quantitative morphometric analysis. Positively stained cells on each slide were counted by morphometric analysis using point counting analysis (14) with a 121-intersection squared grid in the eyepiece of the microscope. Briefly, the percentage of the area of each graft section occupied by cells of a particular antigenic specificity (area infiltrate) was calculated as follows: [number of positive cells under grid intersection/(total number of grid intersections = 121)] $\times$ 100. The graft sections were examined at a magnification of $\times$400. The accuracy of the technique is proportional to the number of points counted. Thus, to maintain a SE of <10%, 15 fields were counted for each labeled section. Results are expressed as the percentage of the area of the tissue section infiltrated by leukocytes (determined with OX1, OX30 labeling) and the phenotypic composition of the infiltrate and subpopulations which are related to the percentage of total leukocytes and are expressed as the percentage of leukocytes.

*Graft_infiltrating cell extraction staining*

Dilacerated hearts were digested with collagenase D (2 mg/ml; Boehringer Mannheim) for 10 min at 37°C. Cells were then collected by extraction through a stainless steel mesh. The resulting suspension was then clarified by Ficoll isolation.

*Quantitative RT-PCR*

Real-time quantitative PCR was performed in an Applied Biosystems GenAmp 7700 Sequence Detection System using SYBR Green PCR Core Reagents (Applied Biosystems, Foster City, CA). The following oligonucleotides were used in this study: rat LAG-3: upper primer is 5'-ATATGAATTCACAGAGGAGATGAGGCAG-3' and lower primer is 5'-ATATGAATTCTCCTGGTCAGAGCTGCCT-3'. Rat INF-g: upper primer is 5'-TGGATGCTATGGAAGGAAAGA-3' and lower primer is 5'-GATTCTGGTGACAGCTGGTG-3'. Rat HPRT: upper primer is 5'-CCTTGGTCAAGCAGTACAGCC-3' and lower primer is 5'-TTCGCTGATGACACAAACATGA-3'. A constant amount of cDNA corresponding to the reverse transcription of 100 ng of total RNA was amplified in 25 $\mu$l of PCR mix containing 300 nM of each primer; 200 $\mu$M dATP, dGTP, dCTP; 400 $\mu$M dUTP; 3 mM $MgCl_2$; 0.25 U of uracyl-N-glycosylase; 0.625 U of AmpliTaq Gold DNA polymerase. The mix was subjected to 40 cycles of amplification. The real-time PCR data were plotted as the $\Delta R_n$ fluorescence signal vs. the cycle number. The $\Delta R_n$ values were calculated by the Applied Biostystems 7700 sequence detection software using the formula: $\Delta R_n = (Rn^+) - (R_n-)$ , where $R_n^+$ is the fluorescence signal of the product at any given time, $R_n$- is the mean fluorescence signal during cycles 3-13 and referred to as the baseline. The $C_t$ value is defined as the cycle number at which the $\Delta R_n$ crosses a threshold. The threshold is set above the background fluorescence to intersect the exponential portion of the amplification curve of a positive reaction. The Ct is inversely proportional to the log amount of template within the PCR.

*Statistical analyses*

Statistical significance was evaluated using aa Mann-Whitney test for the comparison of two groups. Graft survival was evaluated by Kaplan-Meier analysis using the log rank test.

## Results

*LAG-3 mRNA expression in rejected allograft and lymphoid organs*

LAG-3 is expressed by activated T cells in inflamed lymphoid organs and tissues (7). In order to see if LAG-3 is also expressed in rejected allografts, hearts grafts from LEW.1W to LEW.1A rat recipients were analyzed on day 5 (rejection occurring on day 6). Messenger RNA for LAG-3 was analyzed and compared with allografts receiving a tolerance-inducing regiment (anti-CD28 antibodies + CSA, as described (16)) and with isografts. Rejected allografts presented a 7-fold and a 25-fold accumulation of LAG-3 mRNA as compared with tolerated and syngeneic grafts, respectively (Figure 1A). Such an accumulation was not detected in lymph nodes (Figure 1B) or in the spleen of rejecting recipients (Figure 1C).

*Mechanism of action of anti-LAG-3 antibodies*

Anti-LAG-3 antibodies were produced in rabbits by immunization with a synthetic peptide from the extra-loop of LAG-3 Ig-like N-terminal domain , involved in the interaction of LAG-3 with Class II (ref PNAS Huard 1997). Post-immune serum, as well as the IgG fraction, stained <1% of rat spleen cells and 40% of rat spleen cells activated for 48h with ConA, PMA + ionomycin or PHA. Pre-immune serum was negative (data not shown). In order to characterize the effect of anti-LAG-3 antibodies on $LAG-3^+$ cells, complement and ADCC-dependent cytotoxicity was assayed in vitro.

Fifteen % of ConA-activated spleen cells were lysed in the complement-dependent cytotoxicity assay (Figure 2). Given that only 40% of the ConA-activated target cells expressed LAG-3, this assay revealed that about 37 % of the LAG-$3^+$ spleen cells present in the preparation were lysed in vitro as a result of complement activation.

*In vivo*, the depleting activity of anti-LAG-3 antibodies was estimated by measuring the fate of CFSE-labeled activated T cells adoptively transferred to irradiated rat recipients. One day after the injection of therapeutic doses of anti-LAG-3 immune serum, only half the amount of $CFSE^+/CD4^+$ and $CFSE^+/CD8^+$ cells could be recovered from the spleen, as compared with similar injections of pre-immune serum (Figure 3).

*Anti-LAG-3 antibodies delay heart allograft rejection*

From preliminary pharmacokinetic observations, we established that two i.v. injections of 600 μl of anti-LAG-3 rabbit serum on days 0 and 3 resulted in the maintenance of anti-LAG-3 binding activity in recipient's serum for at least 2 weeks. This treatment delayed cardiac allograft rejection from 6 days in untreated and control-treated recipients to a median of 27 days. All recipients, however, eventually rejected their graft within 10 weeks (Figure 4). On day 5, grafts from control-treated recipients were heavily infiltrated by activated T cells and this infiltrate was less important in anti-LAG-3 treated recipients. Infiltration by $CD25^+$ cells and NK cells, however, was not modified by the treatment. Since our anti-LAG-3 antibodies do not recognize LAG-3 in immunohistology, LAG-3 expression by graft infiltrating cells (GICs) was analyzed by flow cytometry after extraction. An average of 8.5 $10^6 \pm 0.76$ GICs could be recovered from control rejected grafts. From heart allografts from anti-LAG-3 treated recipients, only 3.16 $\pm 0.44$ $10^6$ GICs could be recovered (n=3; $p< 0.005$). GICs contained 41.17 $\pm$ 1 % LAG-$3^+$ cells in controls (i.e. 3.5 $10^6$ cells) versus 22.2 $\pm$ 0.9 % in treated animals (i.e. 0.7 $10^6$ cells; n=3; $p<0.0005$; Fig. 5). Analysis of mRNA transcript reinforced these observations that infiltration of heart graft by mononuclear cells was reduced since we measured four times less INFγ mRNA molecules in treated grafts (Figure 5) .

*Anti-LAG-3 antibodies inhibit ongoing acute heart allograft delay rejection*

In order to investigate whether anti-LAG-3 antibodies might serve as a treatment of an ongoing acute allograft rejection, we grafted LEW.1W hearts into LEW.1A allogenic recipients that we maintained untreated over 3 or 4 days. At that time, recipients received an injection of 600 microliter of control or anti-LAG-3 rabbit serum. Control-treated recipients rejected the allografts on day 5 whereas anti-LAG-3 antibodies-treated recipients rejected only on day 11 (Table 1) .

Table 1

| Treatment | Day of rejection | Median survival |
|---|---|---|
| Control serum on day 3 | 5, 5, 5 | 5 |
| Control serum on day 4 | 5, 5, 5 | 5 |
| Anti-LAG-3 serum on day 3 | 12, 13, 9 | 12 (p<0.05 vs. control) |
| Anti-LAG-3 serum on day 4 | 10, 13, 13, 19 | 12.5 (p<0.05 vs. control) |

Table 1: Heart graft recipients were treated on day 3 or 4 with control or anti-LAG-3 antibodies. Rejection was monitored by daily heart palpation.

EXAMPLE 2: Generation of new high affinity hLAG-3 mAbs

Material and methods

Mice were immunized 3 times with hLAG-3-transfected CHO cells ($10^7$ cells, intra-peritoneal injection), followed by a boost *i.v.* injection with 10 μg IMP321, the clinical-grade hLAG-3Ig recombinant protein. Three days after the boost, splenocytes were fused with the X63.AG8653 fusion partner to yield hybridoma cells. Supernatants from hybridomas were screened for their specific binding (FACS analysis) on hLAG-3-tranfected CHO versus wild type (wt) CHO cells.

One murine IgG2a antibody (580.1E9H3A9H12, called A9H12) was selected, subcloned to yield a stable cell line and further characterized for its potency to deplete LAG-$3^+$ cells through CDC (complement-dependent cytotoxicity) and ADCC (antibody-dependent cell-mediated cytotoxicity), given that the murine IgG2a Fc region is known to be the most efficient Fc isotype in mice at delivering these activities, even on heterologous cells (i.e. CHO cells or human PBMCs). Similarly, a second IgM antibody (31G11E8, called 31G11) was also selected.

Results

Dose-dependent binding of A9H12 was first compared to the reference LAG-3-specific 17B4 mAb on hLAG-3-tranfected CHO cells and on LAG-$3^+$ *in vitro* activated human T cells (Figure 6). A9H12 displayed a greater binding than the reference 17B4 mAb on both cell types. For instance, significant binding of A9H12 to activated human T cells was observed with a concentration as low as 0.01 pg/ml.

For CDC testing, the target cells used in this assay were LAG-$3^+$ CHO cells compared to wt CHO cells (Figure 7A). Both types of cells were incubated for 1 hour at 37°C with either A9H12, its murine isotype-matched IgG2a negative control mAb, 31G11, its murine isotype-matched IgM negative control or the reference 17B4 (IgG1) mAb and rabbit serum containing active complement. Cell viability was then assessed using 7-Amino-Actinomycin D (7-AAD), a fluorescent dye labelling cells which have lost their membranous integrity, a phenomenon which appeared rapidly after death. The percentage of 7-AAD-positive CHO cells (i.e. dead target cells) was determined by flow cytometry analysis. A9H12 displayed a potent and specific cytotoxic activity in this CDC assay, killing only LAG-$3^+$ CHO cells in the presence of complement (Figure 7B). The anti-LAG-3 Ab was titered down to determined the efficacy of the antibody to activate CDC at low concentration of antibody. A9H12 efficiently induced LAG-$3^+$ CHO cells killing at a concentration as low as 0.01 pg/ml (Figure 7C). The IgG1 17B4 antibody was also tested in this assay and had no effect (Figure 7D, left panel), showing that not all LAG-3 mAbs could induce cytotoxicity in this bioassay. As observed with A9H12, the second 31G11 LAG-3-specific mAb also induced LAG-$3^+$ CHO cells killing (Figure 7D, right panel).

The CDC bioassay was also performed on PBMCs stimulated with the superantigen SEB. The cytotoxicity of A9H12 and 31G11 were analysed on both activated (namely $CD25^+$/LAG-$3^+$ cells) and non-activated (namely $CD25^-$/LAG-$3^-$ cells) $CD4^+$ helper T and $CD8^+$ cytotoxic T cells. Only activated $CD4^+$ and $CD8^+$ T cells were specifically killed by A9H12 and 31G11 (Figure 7E), showing that activated human T cells are susceptible to A9H12- or 31G11-specific killing in the presence of complement.

For ADCC testing, PMBCs were stimulated for one day with IL-2 to serve as effector cells and LAG-$3^+$ CHO cells were labelled with the vital dye CFSE to serve as target cells. In the presence of A9H12, PBMCs were able to kill a significant percentage of LAG-$3^+$ CHO cells and this effect was increased with the number of effector cells (Figure 8A). In the presence of 17B4, only a small percentage of target cells was killed even at a high E:T ratio (Figure 8A), showing that not all LAG-3 mAbs could induce cytotoxicity in this bioassay. The A9H12 LAG-3 mAb was titered down to determine the efficacy of the antibody to induce ADCC at low concentration of antibody. A9H12 efficiently induced LAG-$3^+$ CHO cells killing at a concentration as low as 0.01 pg/ml (Figure 8B).

EXAMPLE 3: Testing depleting LAG-3 antibodies in a collagen-induced arthritis mouse model

Material and methods

*Animals and materials*

Male DBA/1 (H-$2^q$) mice, 8-10 weeks old, were obtained from Janvier Laboratories. All animal experiments were performed according to local guidelines. BovineCII (joint cartilage) was purchased from BioCol. Incomplete Freund's adjuvant was provided by Sigma. Heat-killed *M. tuberculosis* H37Ra was purchased from Difco Laboratories.

*Induction of collagen-induced arthritis (CIA)*

The induction and assessment of CIA were performed as previously described in two publications (13, 15). Complete freund's adjuvant was prepared by mixing 100 mg heat-killed *Mycobacterium tuberculosis* in 13.3 ml IFA (final concentration 7.5 mg/ml). Bovine CII was dissolved at 3 mg/ml in 10 mM acetic acid overnight at 4°C. An emulsion was formed by mixing 2 volumes of CII with 1 volume of CFA. The CII solution and the emulsion with CFA were always freshly prepared. Male DBA/1 mice were intra-dermally injected at the base of the tail with a total of 100 $\mu$l of emulsion containing 200 $\mu$g CII and 250 $\mu$g *M. tuberculosis* on day 1 (D1). The injection was repeated at day 21 (D21) . As control, three mice were injected with the emulsion of CFA without CII.

*Clinical assessment of arthritis*

Mice were examined for signs of arthritis three times a week from day 22. The disease severity was determined with the following scoring system for each limb: score 0 = normal; score 1 = swelling of footpad or joint; score 2 = swelling of footpad and 1 or 2 joints; score 3 = swelling of footpad and 3 or 4 joints; score 4 = swelling of footpad and all joints. Each paw was graded, and the 4 scores were summed so that the maximum possible score was 16 per mouse. Incidence was expressed as the percentage of mice with an arthritis score $\geq$ 1.

Results

CIA was induced by i.d. injections of bovine type II collagen (CII) emulsified in CFA containing 250 μg *M. tuberculosis.* After one injection, 4 out of 22 mice had developed arthritis at D21. Two weeks after the second injection, at D35, 80-90% of the mice had developed clinical signs of arthritis (Figure 9). The mice exhibited clinical scores covering the full range of responses from 1 to 16 with some limbs showing severe swelling of the footpad, ankle/wrist joint and digits (Table 2). None of the control animals (injected with CFA without CII) developed signs of arthritis (data not shown).

Table 2

| Days | Mean | SEM |
|---|---|---|
| 25 | 2.2 | 0.9 |
| 27 | 2.7 | 0.8 |
| 29 | 5.7 | 1.0 |
| 32 | 9.2 | 1.5 |
| 34 | 10.5 | 1.5 |
| 36 | 10.9 | 1.6 |
| 39 | 10.8 | 1.7 |
| 41 | 10.9 | 1.6 |
| 43 | 11.2 | 1.5 |
| 46 | 11.7 | 1.3 |
| 53 | 13.1 | 1.2 |
| 55 | 13.3 | 1.1 |

Table 2: Mean clinical scores (±SEM) over 55 days. Male DBA/1 mice (n=10) were injected i.d. with bovine type II collagen (200 mg) emulsified in CFA containing 250 mg M. tuberculosis at D1 and D21.

Our results show that with the CIA protocol used, it is possible to obtain a high percentage (80-90%) of mice developing signs of arthritis. This experimental protocol provides a model to evaluate the therapeutic effect of depleting LAG-3 antibodies (specific for mouse LAG-3) in auto-immune diseases.

200 μg of the depleting LAG-3 mAb (A9H12 or 31G11) are injected i.p. or i.v. on day 15 and 25. Both a significant decrease in arthritis incidence and a significant lowering of mean clinical scores are involved.

EXAMPLE 4: Complement-dependent cytotoxicity (CDC) and antibody-dependent cell-mediated cytotoxicity (ADCC) induced by IMP731

Materials and methods

A new murine mAb with depleting properties, A9H12, has been shown to recognize also baboon and macaque monkey LAG-$3^+$ cells with high avidity and had been chosen as our lead depleting therapeutic mAb (ImmuTune IMP731) .

A9H12 has been chimerized with a human IgG1 Fc region using standard genetic engineering and PCR protocols, to give CDC (complement-dependent cytotoxicity) and ADCC (antibody-dependent cell cytotoxicity) properties.

The A9H12 VH and VL cDNA sequences derived from A9H12 hybridoma cell mRNA were fused upstream of the human CH1-hinge-CH2-CH3 IgG1 domains and Ckappa chains, respectively (Figure 10) .

The two light and heavy IMP731 chimeric chains were independently cloned into separate expression plasmids (Figure 11 panel A and B, respectively) under the control of the PGK (or SRalpha in another construction, not shown) promoter. These 2 plasmids were cotransfected (transitory transfection) together into CHO cells and IMP731 was purified from the culture supernatant at day 2 or 3 by using protein A column affinity capture and elution at pH 3. After neutralisation with Tris-HCl the purified IMP731 antibody was tested in CDC and ADCC experiments for its ability to kill LAG-$3^+$ target cells.

The two IMP731 light and heavy chains were then cloned together with the PGK (or SRalpha, not shown) promoter in a head-to-tail situation for coordinated expression of the two IMP731 chains from the same integration site (Figure 12). This bi-cistronic IMP731 expression plasmid was used for stable transfection and selection of high-productivity (e.g. more than 20 picogramme IMP731 protein per million CHO-S cells per day) CHO-S cells using increasing concentrations of hygromycine in serum-free medium.

Results

Dose-dependent binding of IMP731 was first assessed on hLAG-3-tranfected CHO cells (Figure 13A) and on LAG-$3^+$ *in vitro* activated human T cells (Figure 13B). IMP731 displayed a significant binding on both cell types with a concentration as low as 0.01 pg/ml for activated T cells.

For complement dependent cytotoxicity (CDC) testing, the target cells used in this assay were LAG-$3^+$ CHO cells (Figure 14). Cells were incubated either with IMP731 or its human isotype-matched IgG1 negative control and then with rabbit serum containing active complement for 1 hour at 37°C. Cell viability was then assessed using 7-Amino-Actinomycin D (7-AAD). 7-AAD is a fluorescent dye labelling cells which have lost their membranous integrity, a phenomenon which appeared rapidly after death. The percentage of 7-AAD-positive CHO cells (i.e. dead target cells) was determined by flow cytometry analysis. IMP731 displayed a potent and specific cytotoxic activity in this CDC assay, killing only LAG-$3^+$ CHO cells in the presence of complement (Figure 14).

For antibody-dependent cell-mediated cytotoxicity (ADCC) testing, PBMCs were stimulated for one day with IL-2 to serve as effector cells and LAG-$3^+$ CHO cells were labelled with the vital dye CFSE to serve as target cells. In the presence of IMP731, PBMCs were able to kill a high percentage of LAG-$3^+$ CHO cells (Figure 15A). IMP731 LAG-3 Ab was tittered down to determine the efficacy of the antibody to induce ADCC at low concentration of antibody. IMP731 significantly induced LAG-$3^+$ CHO cells killing at a concentration as low as 0.01 pg/ml (Figure 15B). LAG-$3^+$ but not LAG-$3^-$ cells were killed by the addition of IMP731 in this assay (Figure 15C).

It appeared that binding and functional activities of IMP371 were similar to the parental A9H12 murine mAb produced by hybridoma cells.

**References**

1. Waldmann H. The new immunosuppression: just kill the T cell. Nat Med 2003; 9 (10): 1259.
2. Monk NJ, Hargreaves RE, Marsh JE, et al. Fc-dependent depletion of activated T cells occurs through CD40L-specific antibody rather than costimulation blockade. Nat Med 2003; 9 (10): 1275.
3. Andre P, Prasad KS, Denis CV, et al. CD40L stabilizes arterial thrombi by a beta3 integrin--dependent mechanism. Nat Med 2002; 8 (3): 247.
4. Avice MN, Sarfati M, Triebel F, Delespesse G, Demeure CE. Lymphocyte activation gene-3, a MHC class II ligand expressed on activated T cells, stimulates TNF-alpha and IL-12 production by monocytes and dendritic cells. J. Immunol. 1999; 162: 2748.
5. Andreae S, Piras F, Burdin N, Triebel F. Maturation and activation of dendritic cells induced by lymphocyte activation gene-3 (CD223). J Immunol 2002; 168 (8): 3874.
6. Andreae S, Buisson S, Triebel F. MHC class II signal transduction in human dendritic cells induced by a natural ligand, the LAG-3 protein (CD223). Blood 2003; 102 (6): 2130.
7. Triebel F. LAG-3: a regulator of T-cell and DC responses and its use in therapeutic vaccination. Trends Immunol 2003; 24 (12): 619.
8. Macon-Lemaitre L, Triebel F. The negative regulatory function of the lymphocyte-activation gene-3 co-receptor (CD223) on human T cells. Immunology 2005; 115 (2): 170.
9. Kohler and Milstein. Continuous cultures of fused cells secreting antibody of predefined specificity. Nature. 1975; 256(5517): 495-497.
10. Kozbor et al., 1985, in Monoclonal Antibodies and Cancer Therapy, Alan R. Liss, Inc., pp. 77-96.
11. Ono et al, Improved technique of heart transplantation in rats. J. Thorac. Cardiovasc. Surg. 57, 225-229.
12. Stasiuk et al, Collagen-induced arthritis in DBA/1 mice: Cytokine gene activation following immunization with type II collagen. Cellular Immunol. 1996; 173: 269-275.
13. Campbell et al, Collagen-induced arthritis in C57BL/6 (H-2b) mice: new insights into an important disease model of rheumatoid arthritis. Eur. J. Immunol. 2000; 30: 1568-1575.
14. Inglis et al, Collagen-induced arthritis in C57BL/6 is associated with a robust and sustained T-cell response to type II collagen. Arthritis Research & Therapy. 2007; 9: R113.
15. Hiroaki et al, A tumor necrosis factor receptor loop peptide mimic inhibits bone destruction to the same extent as anti-tumor necrosis factor monoclonal ntibody in lurine collagen-induced arthritis. arthritis & Rheumatism. 2007; 56(4): 1164-1174.

## Claims

1. A cytotoxic anti-LAG-3 monoclonal antibody or fragment thereof for use in treating or preventing organ transplant rejection or treating an autoimmune disease, wherein said antibody or fragment thereof comprises a human IgG1 or IgM or murine IgG2a subclass Fc fragment and a Fab fragment which binds LAG-3 protein, and wherein said antibody or fragment thereof causes depletion of LAG-$3^+$ activated T cells via complement-dependent cytotoxicity (CDC) and/or antibody-dependent cell cytotoxicity (ADCC).

2. A cytotoxic anti-LAG-3 monoclonal antibody or fragment thereof for use according to claim 1, wherein depletion of LAG-$3^+$ activated T cells is measured by changes in peripheral blood lymphocyte numbers.

3. A cytotoxic anti-LAG-3 monoclonal antibody or fragment thereof for use according to claim 1 or claim 2, wherein said monoclonal antibody or fragment thereof is capable of causing depletion of more than 30% preferably more than 50 % of LAG-$3^+$ activated T cells.

4. A cytotoxic anti-LAG-3 monoclonal antibody or fragment thereof for use according to any one of claims 1 to 3, wherein said monoclonal antibody or fragment thereof exhibits (i) more than 50 % of maximal CDC activity at a mAb concentration of less than 0.1 $\mu$g/ml or (ii) more than 50% of maximal ADCC activity at a mAb concentration of less than 0.1 $\mu$g/ml.

## Patentansprüche

1. Zytotoxischer monoklonaler anti-LAG-3-Antikörper oder Fragment davon zur Anwendung in der Behandlung oder Prävention von Organtransplantabstoßung oder in der Behandlung einer Autoimmunerkrankung, wobei der Antikörper oder das Fragment davon ein Fc-Fragment der menschlichen IgGl oder IgM oder murinen IgG2a Unterklasse und ein Fab-Fragment umfasst, das ein LAG-3-Protein bindet, und wobei der Antikörper oder das Fragment davon eine Depletion von LAG-$3^+$ aktivierten T-Zellen über Komplement-abhängige Zytotoxizität (CDC) und/oder Antikörper-abhängige Zell-Zytotoxizität (ADCC) verursacht.

2. Zytotoxischer monoklonaler anti-LAG-3-Antikörper oder Fragment davon zur Anwendung gemäß Anspruch 1, wobei die Depletion von LAG-$3^+$ aktivierten T-Zellen durch Veränderungen der Lymphozytenzahlen im peripheren Blut gemessen wird.

3. Zytotoxischer monoklonaler anti-LAG-3-Antikörper oder Fragment davon zur Anwendung gemäß Anspruch 1 oder 2, wobei der monoklonale Antikörper oder das Fragment davon in der Lage ist, eine Depletion von mehr als 30% vorzugsweise mehr als 50% der LAG-$3^+$ aktivierten T-Zellen zu verursachen.

4. Zytotoxischer monoklonaler anti-LAG-3-Antikörper oder Fragment davon zur Anwendung gemäß irgendeinem der Ansprüche 1 bis 3, wobei der monoklonale Antikörper oder das Fragment davon (i) mehr als 50% der maximalen CDC-Aktivität bei einer mAb-Konzentration von weniger als 0,1 pg/ml oder (ii) mehr als 50% der maximalen ADCC-Aktivität bei einer mAb-Konzentration von weniger als 0,1 pg/ml zeigt.

## Revendications

1. Anticorps monoclonal cytotoxique anti-LAG-3 ou fragment de celui-ci pour le traitement ou la prévention d’un rejet de greffon d’organe ou le traitement d’une maladie auto-immune, dans lequel ledit anticorps ou fragment de celui-ci comprend un fragment Fc de sous-classe IgG1 ou IgM humaine ou IgG2a murine et un fragment Fab qui se lie à la protéine LAG-3, et dans lequel ledit anticorps ou fragment de celui-ci provoque une déplétion des cellules T activées LAG-$3^+$ par le biais d’une cytotoxicité dépendante du complément (CDC) et/ou d’une cytotoxicité cellulaire dépendante des anticorps (ADCC).

2. Anticorps monoclonal cytotoxique anti-LAG-3 ou fragment de celui-ci pour utilisation selon la revendication 1, dans lequel la déplétion des cellules T activées LAG-$3^+$ est mesurée par des changements dans le nombre de lymphocytes de sang périphérique.

3. Anticorps monoclonal cytotoxique anti-LAG-3 ou fragment de celui-ci à utiliser selon la revendication 1 ou la revendication 2, dans lequel ledit anticorps monoclonal ou fragment de celui-ci est capable de provoquer une déplétion de plus de 30 %, de préférence de plus de 50 %, des cellules T activées LAG-$3^+$.

**4.** Anticorps monoclonal cytotoxique anti-LAG-3 ou fragment de celui-ci utilisé selon l'une quelconque des revendications 1 à 3, dans lequel ledit anticorps monoclonal ou fragment de celui-ci présente (i) plus de 50 % de l'activité CDC maximale à une concentration mAb inférieure à 0,1 μg/ml ou (ii) plus de 50 % de l'activité ADCC maximale à une concentration mAb inférieure à 0,1 μg/ml.

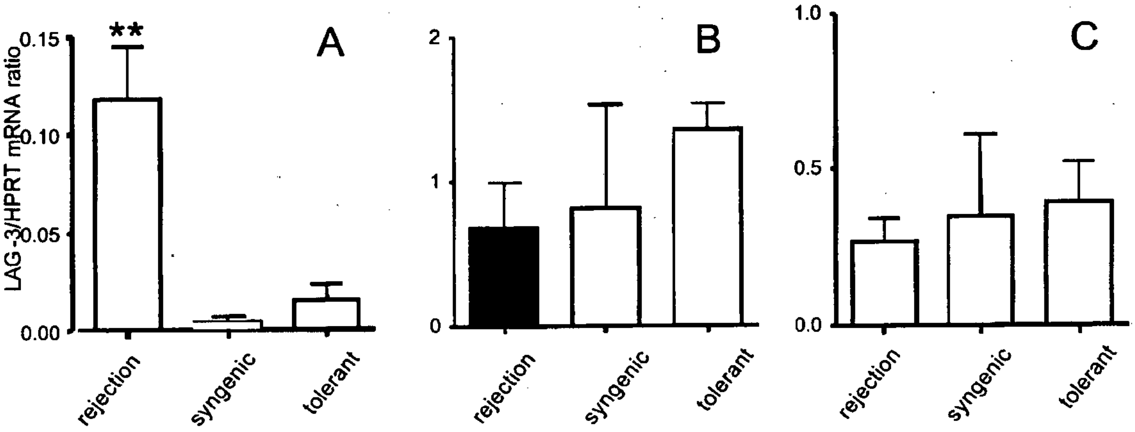


Figure 1

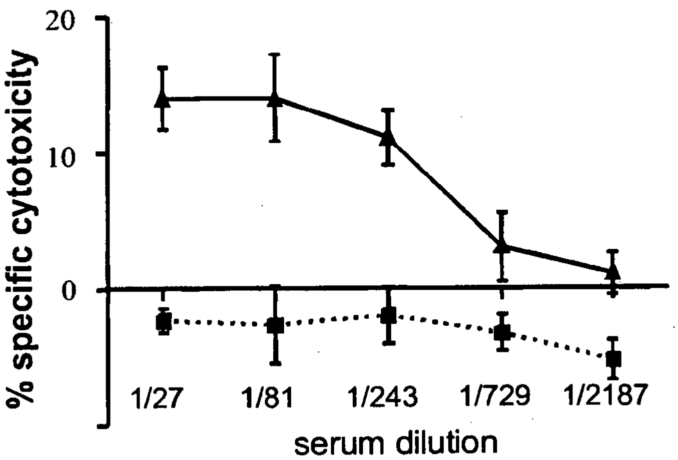


Figure 2

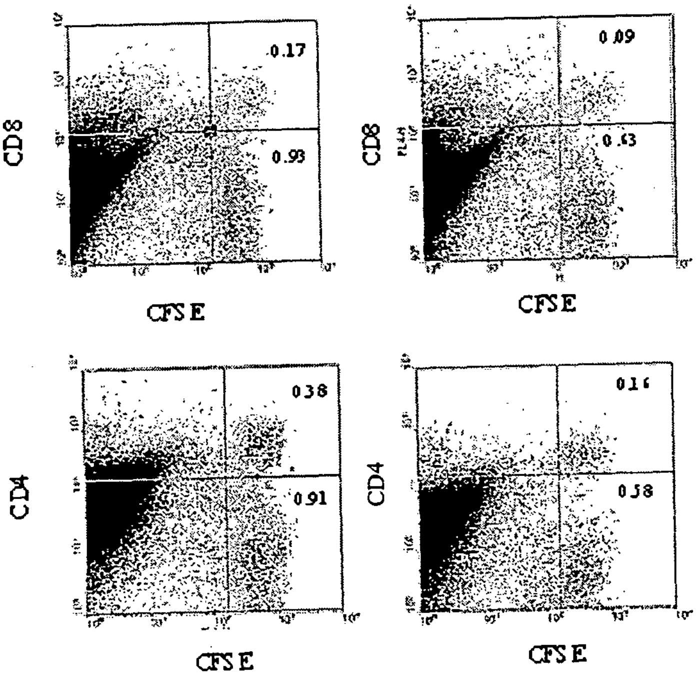


Figure 3

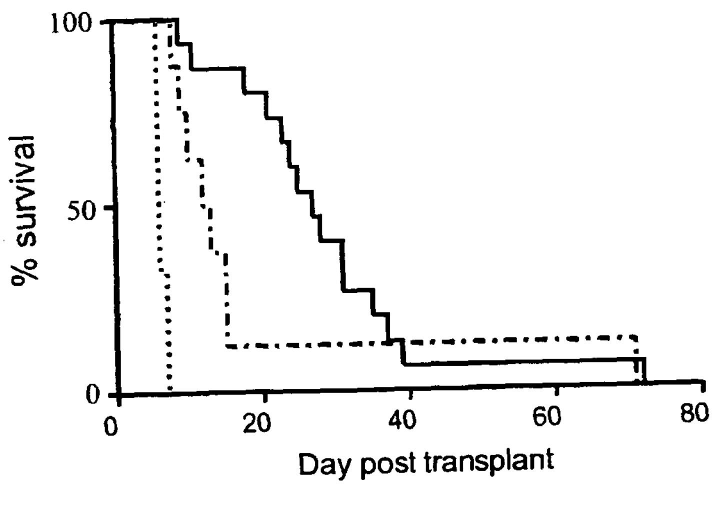


Figure 4

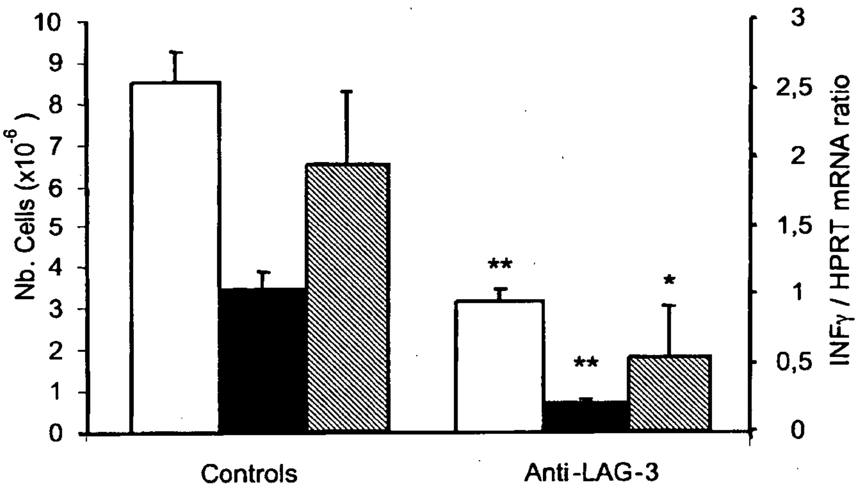


Figure 5

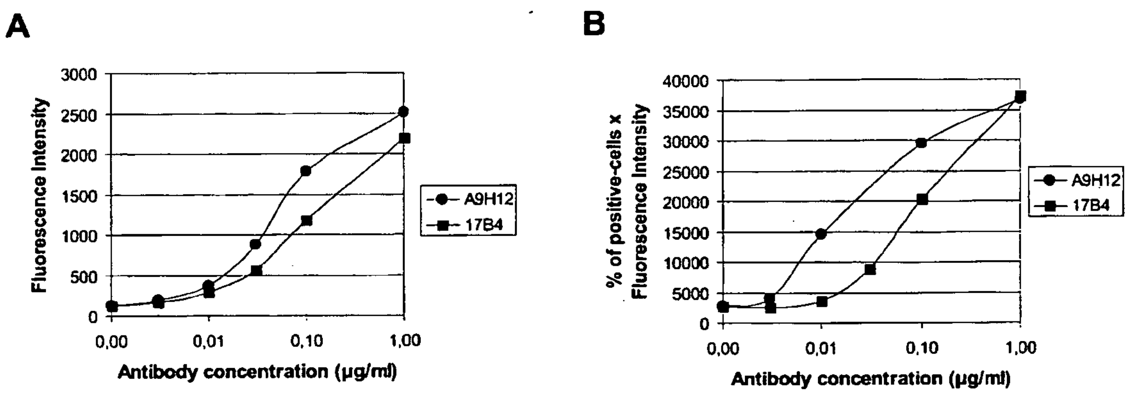


Figure 6

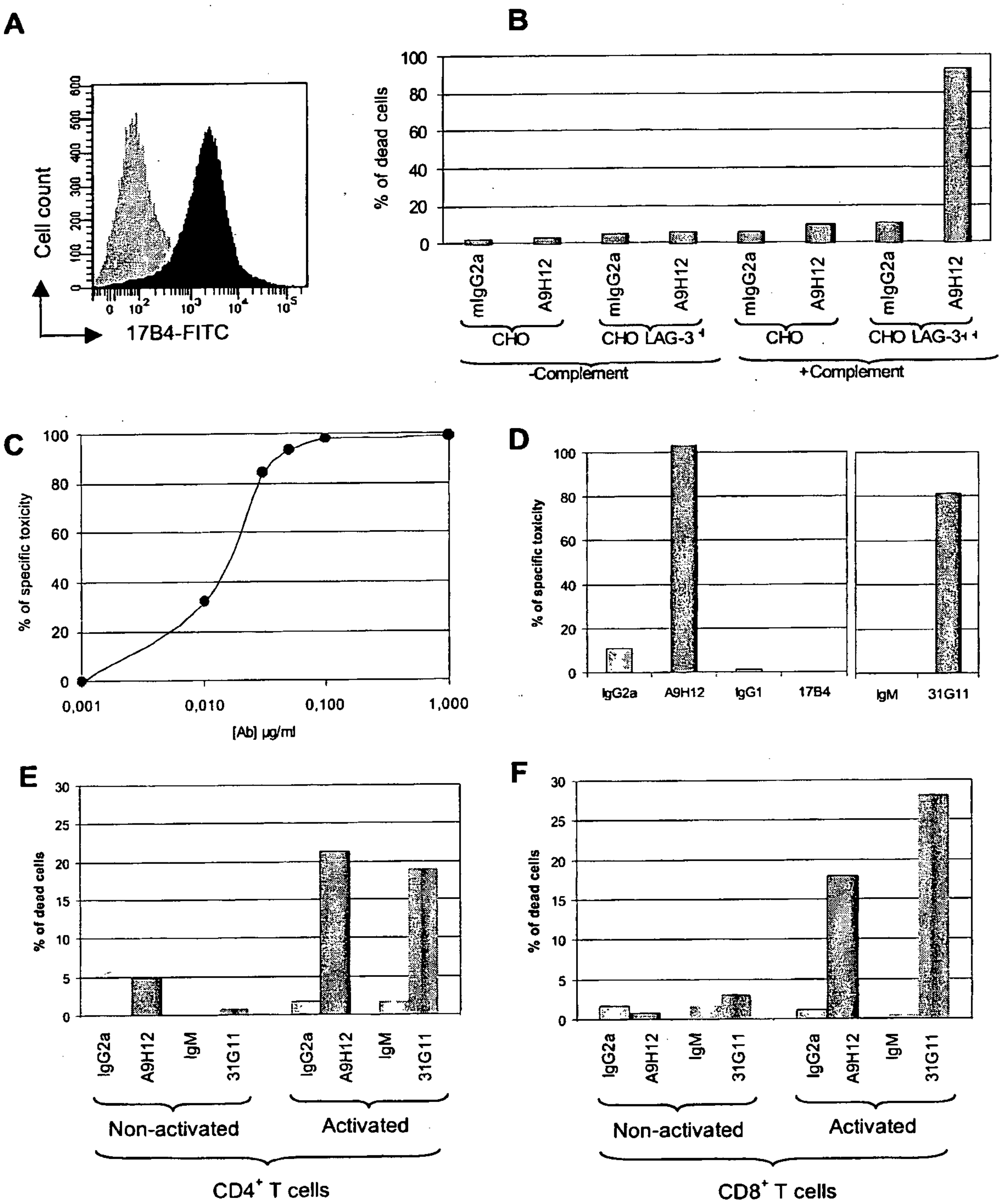
A
Cell count
17B4-FITC
B
% of dead cells
mIgG2a
A9H12
CHO
CHO LAG-3+
-Complement
+Complement
C
% of specific toxicity
0,001
0,010
0,100
1,000
[Ab] µg/ml
D
% of specific toxicity
IgG2a
A9H12
IgG1
17B4
IgM
31G11
E
% of dead cells
IgG2a
A9H12
IgM
31G11
Non-activated
Activated
CD4+ T cells
F
% of dead cells
CD8+ T cells

Figure 7

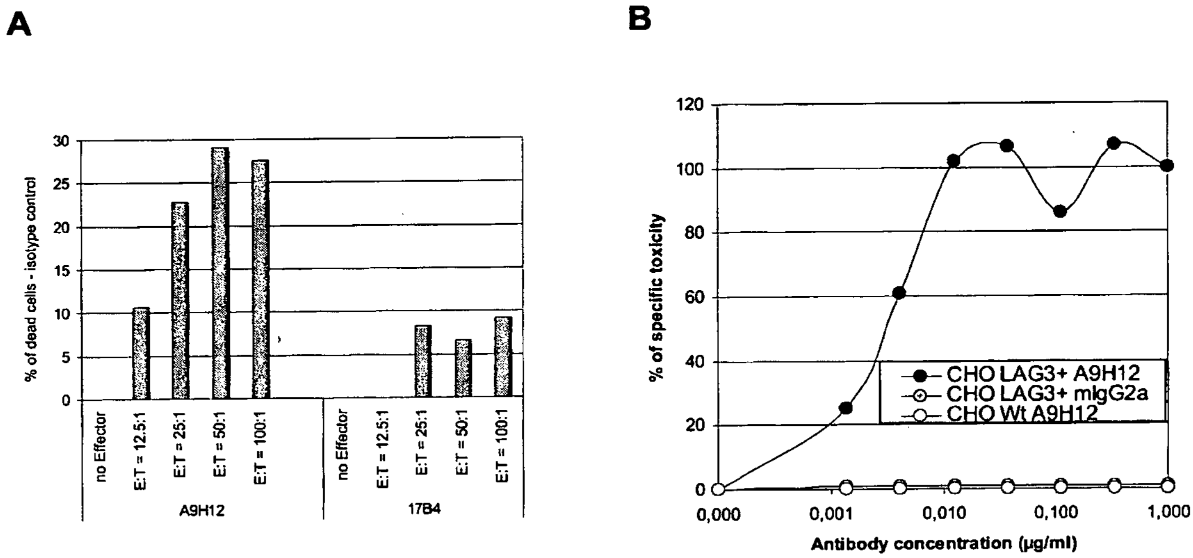


Figure 8

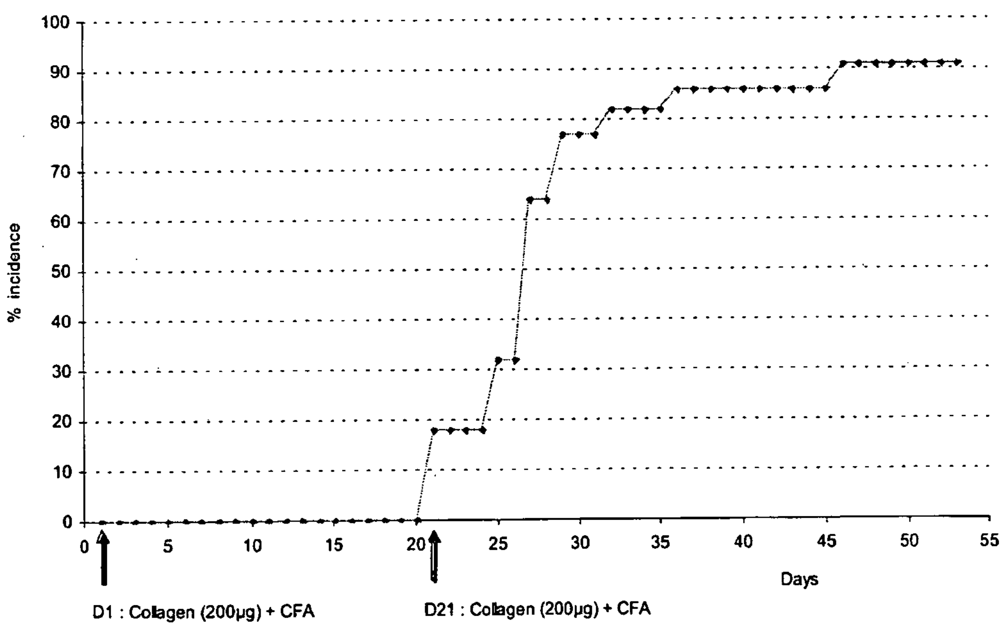


Figure 9

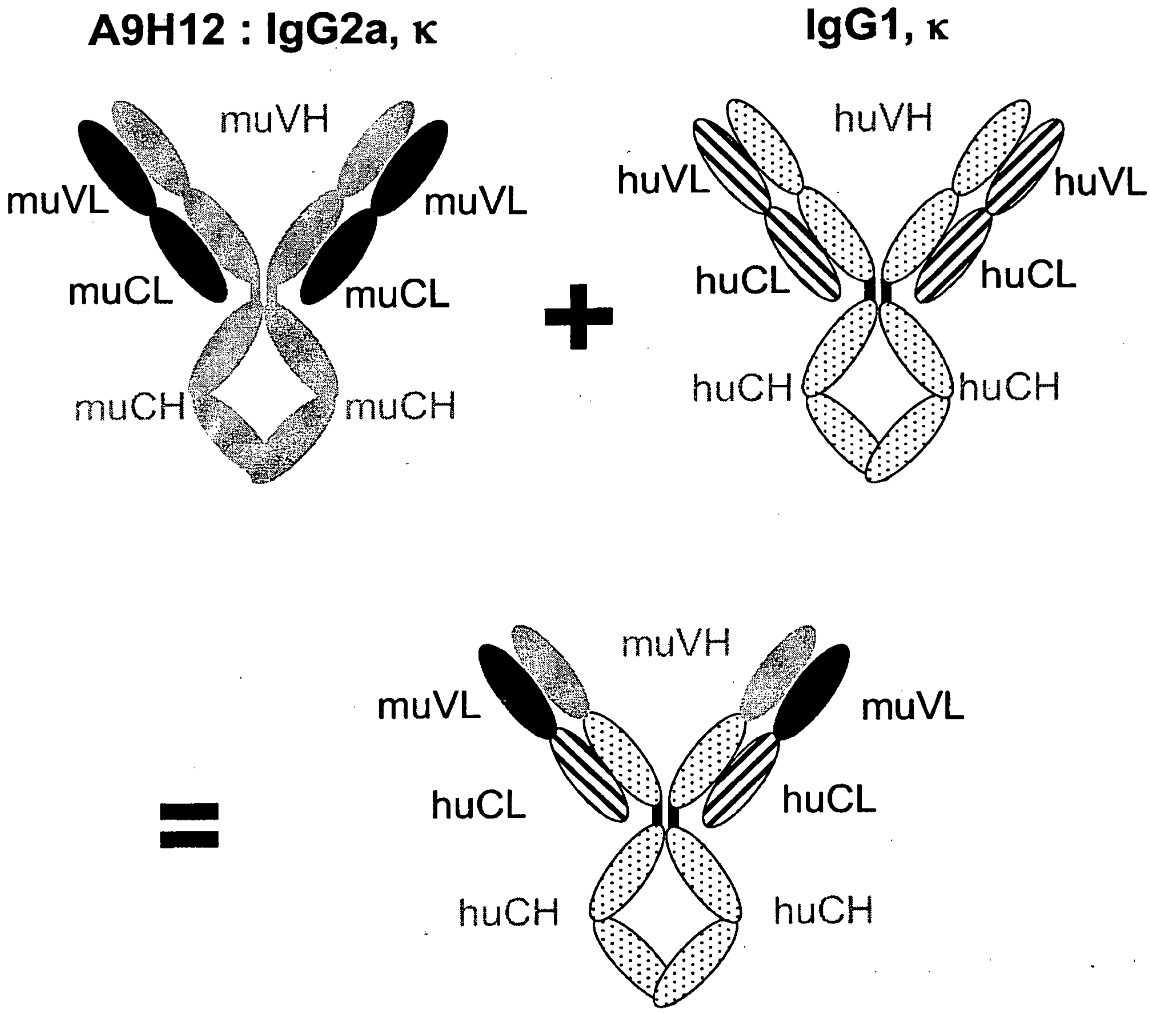
A9H12 : IgG2a, κ
IgG1, κ
muVH
muVL
muVL
muCL
muCL
muCH
muCH
huVH
huVL
huVL
huCL
huCL
huCH
huCH
muVH
muVL
muVL
huCL
huCL
huCH
huCH

Figure 10

A

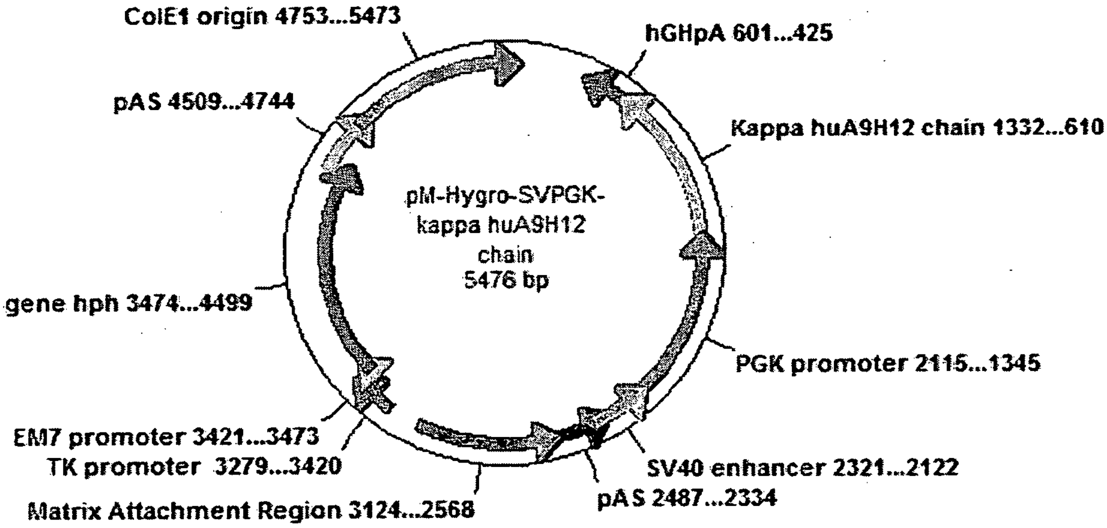
ColE1 origin 4753...5473
hGHpA 601...425
pAS 4509...4744
Kappa huA9H12 chain 1332...610
pM-Hygro-SVPGK-
kappa huA9H12
chain
5476 bp
gene hph 3474...4499
PGK promoter 2115...1345
EM7 promoter 3421...3473
TK promoter 3279...3420
SV40 enhancer 2321...2122
pAS 2487...2334
Matrix Attachment Region 3124...2568

B

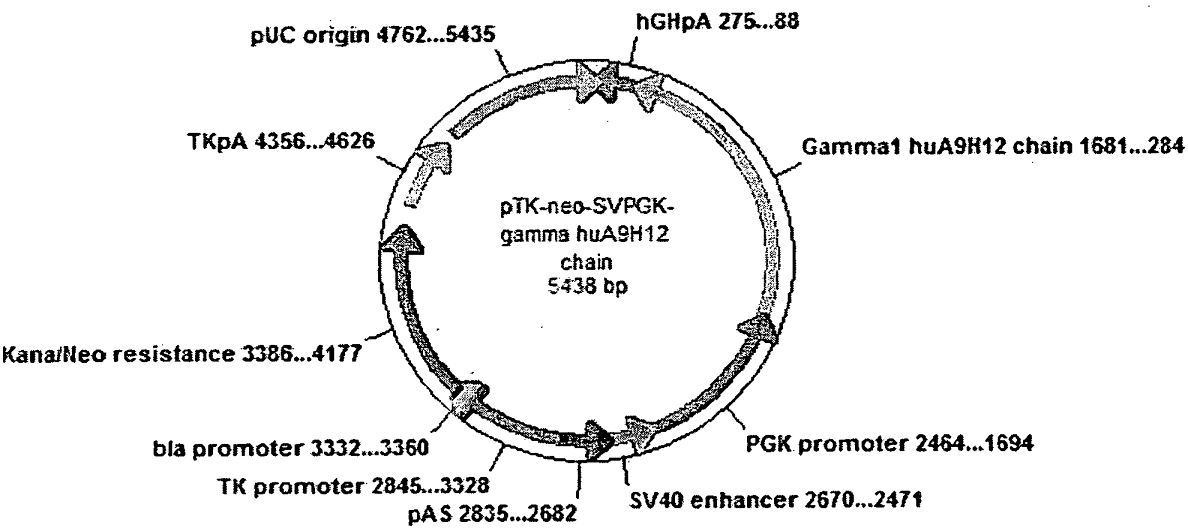
pUC origin 4762...5435
hGHpA 275...88
TKpA 4356...4626
Gamma1 huA9H12 chain 1681...284
pTK-neo-SVPGK-
gamma huA9H12
chain
5438 bp
Kana/Neo resistance 3386...4177
bla promoter 3332...3360
PGK promoter 2464...1694
TK promoter 2845...3328
pAS 2835...2682
SV40 enhancer 2670...2471

Figure 11

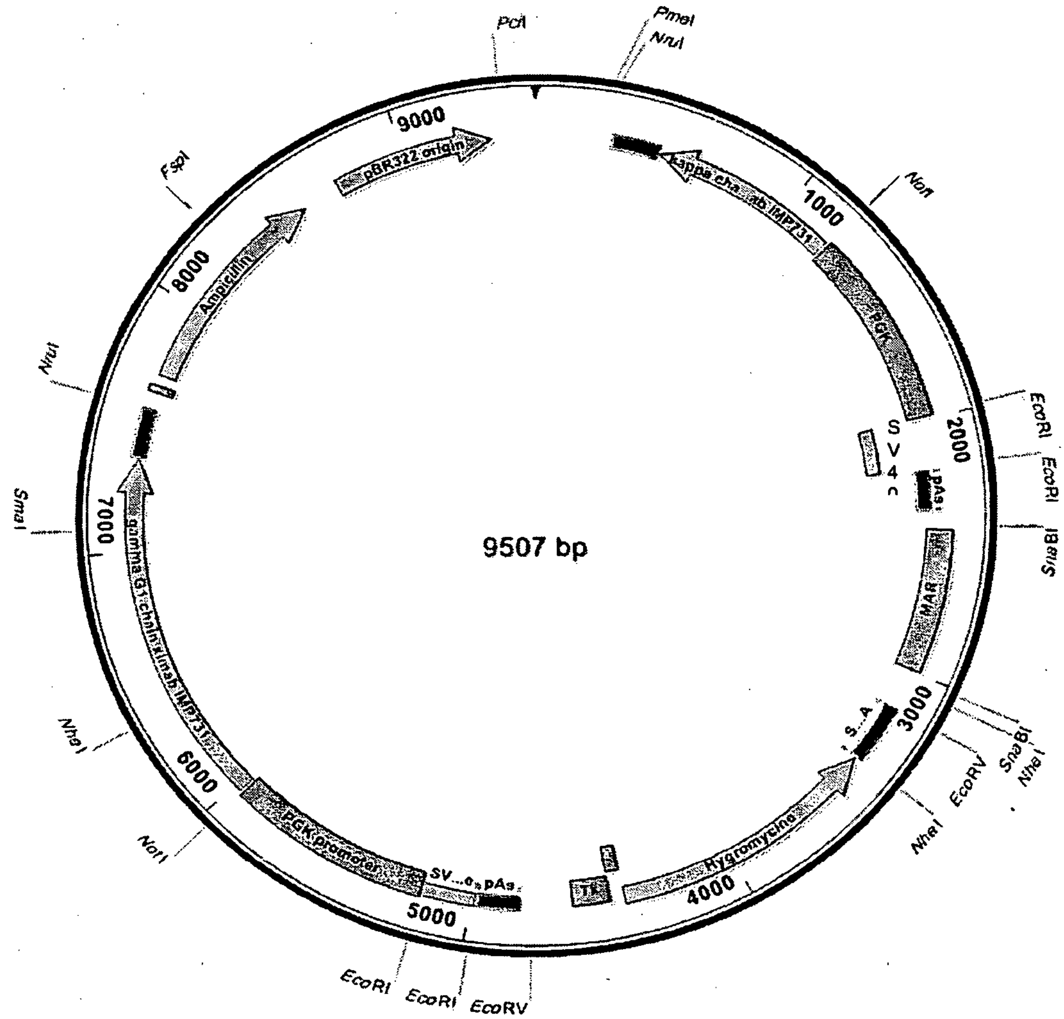
PciI
PmeI
NruI
9000
pBR322 origin
kappa cha...ab IMP731
1000
NotI
FspI
8000
Ampicillin
PGK
NruI
EcoRI
2000
EcoRI
SmaI
7000
9507 bp
pAs
SmaI
MAR
gamma G1 chain ... mab IMP731
3000
SnaBI
NheI
NheI
6000
EcoRV
NheI
NotI
PGK promoter
SV...0 pAs
Hygromycin
4000
TK
5000
EcoRI
EcoRI
EcoRV

Figure 12

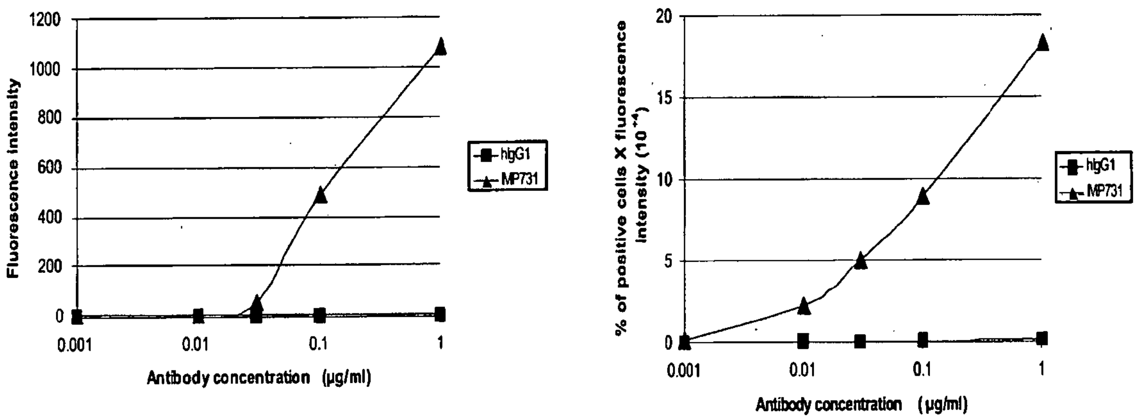


Figure 13

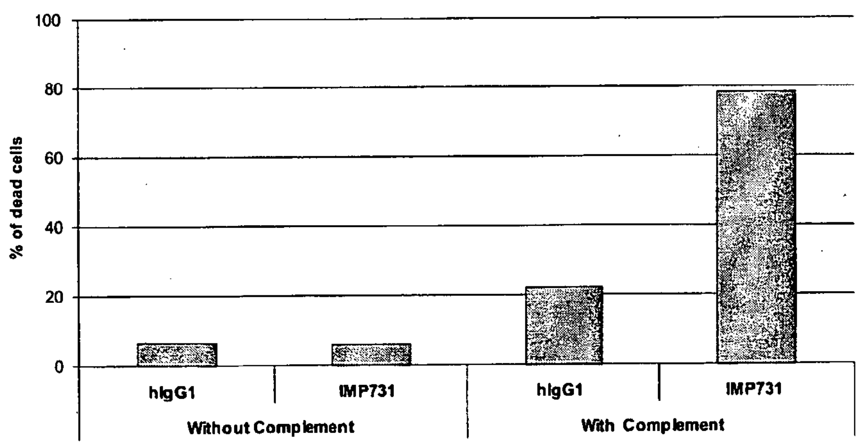


Figure 14

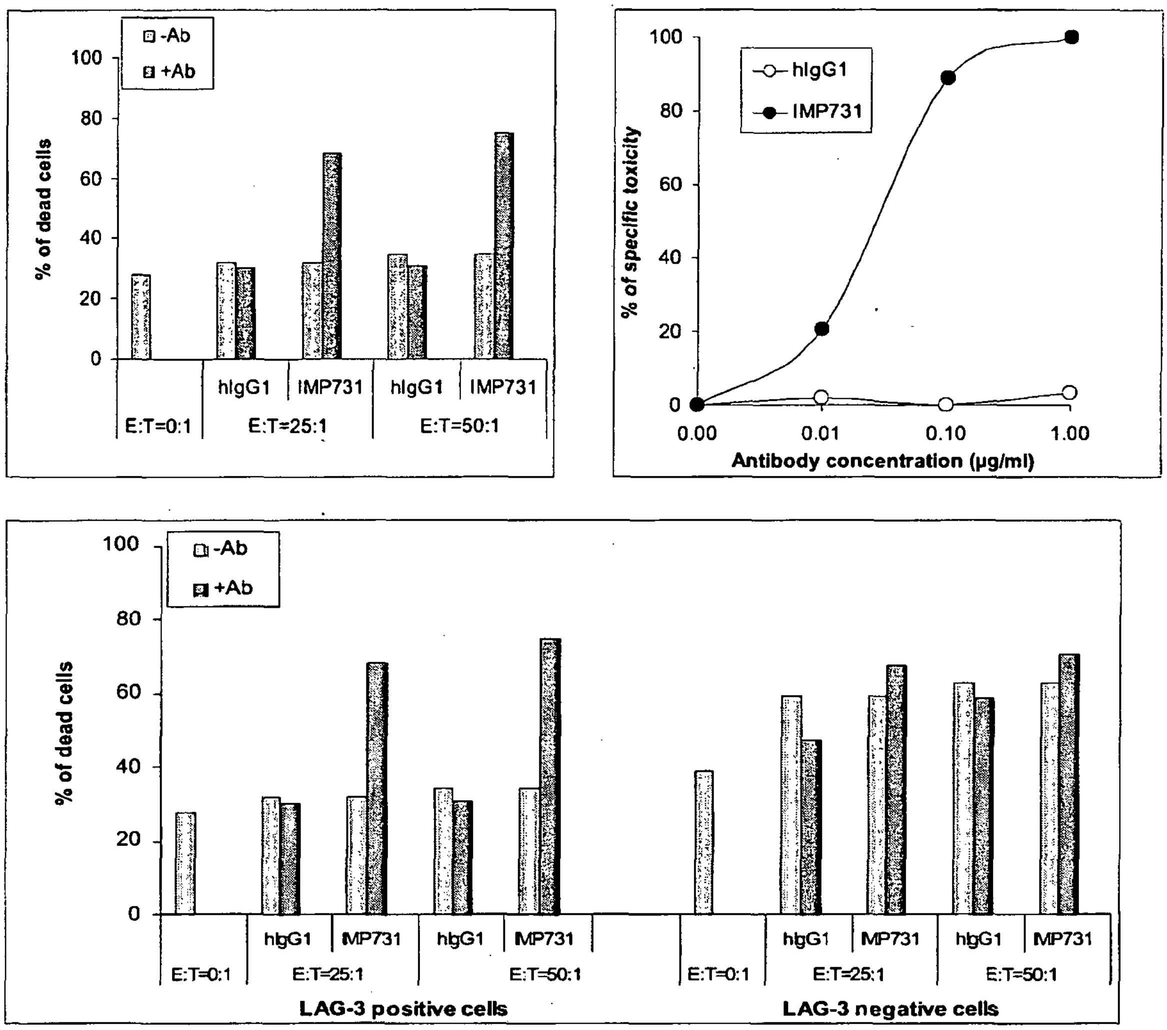
-Ab
+Ab
% of dead cells
100
80
60
40
20
0
hIgG1
IMP731
hIgG1
IMP731
E:T=0:1
E:T=25:1
E:T=50:1
hIgG1
IMP731
% of specific toxicity
0.00
0.01
0.10
1.00
Antibody concentration (µg/ml)
LAG-3 positive cells
LAG-3 negative cells

Figure 15

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2004078928 A
- US 4946778 A


### Non-patent literature cited in the description


- **MONK et al.** *Nature Medicine,* 14 September 2003, vol. 9 (10
- **KISIELOW et al.** *European Journal of Immunology,* 01 July 2005, vol. 35 (7), 2081-2088
- **WALDMANN H.** The new immunosuppression: just kill the T cell. *Nat Med,* 2003, vol. 9 (10), 1259
- **MONK NJ ; HARGREAVES RE ; MARSH JE et al.** Fc-dependent depletion of activated T cells occurs through CD40L-specific antibody rather than costimulation blockade. *Nat Med,* 2003, vol. 9 (10), 1275
- **ANDRE P ; PRASAD KS ; DENIS CV et al.** CD40L stabilizes arterial thrombi by a beta3 integrin--dependent mechanism. *Nat Med,* 2002, vol. 8 (3), 247
- **AVICE MN ; SARFATI M ; TRIEBEL F ; DELESPESSE G ; DEMEURE CE.** Lymphocyte activation gene-3, a MHC class II ligand expressed on activated T cells, stimulates TNF-alpha and IL-12 production by monocytes and dendritic cells. *J. Immunol.,* 1999, vol. 162, 2748
- **ANDREAE S ; PIRAS F ; BURDIN N ; TRIEBEL F.** Maturation and activation of dendritic cells induced by lymphocyte activation gene-3 (CD223). *J Immunol,* 2002, vol. 168 (8), 3874
- **ANDREAE S ; BUISSON S ; TRIEBEL F.** MHC class II signal transduction in human dendritic cells induced by a natural ligand, the LAG-3 protein (CD223). *Blood,* 2003, vol. 102 (6), 2130
- **TRIEBEL F.** LAG-3: a regulator of T-cell and DC responses and its use in therapeutic vaccination. *Trends Immunol,* 2003, vol. 24 (12), 619
- **MACON-LEMAITRE L ; TRIEBEL F.** The negative regulatory function of the lymphocyte-activation gene-3 co-receptor (CD223) on human T cells. *Immunology,* 2005, vol. 115 (2), 170
- **KOHLER ; MILSTEIN.** Continuous cultures of fused cells secreting antibody of predefined specificity. *Nature,* 1975, vol. 256 (5517), 495-497
- **KOZBOR et al.** Monoclonal Antibodies and Cancer Therapy. Alan R. Liss, Inc, 1985, 77-96
- **ONO et al.** Improved technique of heart transplantation in rats. *J. Thorac. Cardiovasc. Surg.,* vol. 57, 225-229
- **STASIUK et al.** Collagen-induced arthritis in DBA/1 mice: Cytokine gene activation following immunization with type II collagen. *Cellular Immunol.,* 1996, vol. 173, 269-275
- **CAMPBELL et al.** Collagen-induced arthritis in C57BL/6 (H-2b) mice: new insights into an important disease model of rheumatoid arthritis. *Eur. J. Immunol.,* 2000, vol. 30, 1568-1575
- **INGLIS et al.** Collagen-induced arthritis in C57BL/6 is associated with a robust and sustained T-cell response to type II collagen. *Arthritis Research & Therapy,* 2007, vol. 9, R113
- **HIROAKI et al.** A tumor necrosis factor receptor loop peptide mimic inhibits bone destruction to the same extent as anti-tumor necrosis factor monoclonal ntibody in lurine collagen-induced arthritis. *arthritis & Rheumatism,* 2007, vol. 56 (4), 1164-1174